# EUROPEAN PATENT APPLICATION

(11) **EP 4 729 064 A1**
(43) Date of publication of application: **22.04.2026**
(21) Application number: 26163298.8
(22) Date of filing: 03.05.2019
(51) Int. Cl.: A61K 35/33, A61K 35/28, A61P 19/02, A61L 27/38, C12N 5/077, A61K 31/16, A61K 31/167, A61K 31/19, A61K 31/26, A61K 31/706

(54) **DE-DIFFERENTIATED FIBROBLAST-CONDITIONED MEDIA FOR TREATING OR PREVENTING DISC DEGENERATION**

(30) Priority: 04.05.2018 US 201862666777 P
(62) Divisional of application: 19795797.0
(71) Applicant: FibroBiologics, Inc., Houston, Texas 77289 (US)
(72) Inventor: O'HEERON, Pete, Houston, 77059 (US); ICHIM, Thomas, Houston, 77058 (US)
(74) Representative: J A Kemp LLP

(57) **Abstract**

Embodiments of the disclosure include methods and compositions for disc repair in a mammal using conditioned media (and/or one or more components therefrom) from fibroblasts that have been de-differentiated and cultured optionally with one or more particular conditions and/or compositions. In specific cases, fibroblasts that have been de-differentiated are exposed to hypoxia, histone deacetylase inhibitor(s), DNA methyltransferase inhibitor(s), or a combination thereof, and the conditioned media therefrom is provided in an effective amount to an individual.

## Description

This application claims priority to U.S. Provisional Patent Application Serial No. 62/666,777, filed May 4, 2018, which is incorporated by reference herein in its entirety.

### TECHNICAL FIELD

Embodiments of the disclosure concern at least the technical fields of cell biology, molecular biology, biochemistry, and medicine.

### BACKGROUND

It is well known that the intervertebral discs are made of highly organized matrices of collagen, water, and proteoglycans. Proteoglycan production in the discs is believed to occur by differentiated chondrocytes. Each intervertebral disc comprises a central highly hydrated and gelatinous nucleus pulposus (nucleus) surrounded by an elastic and highly fibrous annulus fibrosus (annulus). Cartilaginous endplates provide a connection to the vertebrae inferiorly and superiorly to the intervertebral disc. This cushioned arrangement within the intervertebral discs allows the discs to facilitate movement and flexibility within the spine while dissipating hydraulic pressure through the spine.

During aging, mechanical stress, and/or as a result of other environment and/or genetic changes, the intervertebral disc may begin to degenerate. It is known that with aging, the matrix of the disc undergoes substantial structural, molecular, and mechanical changes, including a loss in the demarcation between the annulus fibrosus (AF) and the nucleus pulposus (NP) [1, 2], alterations in collagen content, and a decrease in proteoglycan [3], resulting in loss of structural integrity, decreased hydration, and an inability to withstand load [4]. Because matrix changes largely reflect alterations in the biology of the cells, it is not surprising to find that during aging and degeneration, the cells of the NP exhibit altered patterns of gene and protein expression for matrix molecules, degrading enzymes, and catabolic cytokines [5-7].

In many cases, the degeneration of the disc is associated with death of nucleus pulposus cells. For example, in one study it was shown that with increasing compressive stress on the disc, the inner and middle annulus became progressively more disorganized, and the percentage of cells undergoing apoptosis increased. Stress also caused expression of Type II collagen to be suppressed, whereas the expression of aggrecan decreased at the highest stress levels in apparent proportion to the decreased nuclear cellularity. Compression for one week did not affect the disc bending stiffness or strength but did increase the neutral zone by 33%. As suggested by the finite-element model, during sustained compression, tension is maintained in the outer annulus and lost in the inner and middle regions where the hydrostatic stress was predicted to increased nearly 10-fold. Discs loaded at the lowest stress recovered annular architecture but not cellularity after one month of recuperation. Discs loaded at the highest stress did not recover annular architecture, displaying islands of cartilage cells in the middle annulus at sites previously populated by fibroblasts [8]. Another study investigated whether fractures of the vertebrae induce apoptosis in the affected disc tissue from patients (n = 17) undergoing open reduction and internal fixation of thoracolumbar spine fractures. In contrast to healthy control disc tissues, samples from traumatic thoracolumbar discs showed positive TUNEL staining and a significant increase of caspase-3/7 activity. Interestingly, analyses of the initiator caspase-8 and - 9 revealed significantly increased activation levels compared to control values, suggesting the coexistent activation of both the extrinsic (receptor-mediated) and intrinsic (mitochondria-mediated) apoptosis pathway. Accordingly, expression levels of the Fas receptor (FasR) mRNA were significantly increased. Although the TNF receptor I (TNFR I) was only slightly upregulated, corresponding TNF-alpha from trauma affected discs presented significantly increased mRNA expression values. Furthermore, traumatic disc cells demonstrated significantly reduced expression of the mitochondria-bound anti-apoptotic Bcl-2, thereby maintaining baseline transcriptional levels of the pro-apoptotic Bax protein when compared to control disc cells [9]. Numerous studies have shown death of nucleus pulposus cells to be associated with progression of disc degeneration [10-12]. One molecular mechanism identified to be implicated in death of nucleus pulposus cells is the death receptor Fas and its cognate receptor Fas Ligand [13-15]. Other molecules have also been implicated including DR4 [16, 17], TNF-alpha [18-20], ADAMTS-7 and ADAMTS-12 [21, 22], and IL-2 [23].

Intradiscal injections for prevention of nucleus pulposus cell apoptosis were previously performed using a variety of agents, including gene therapy, stem cell therapy, and growth factors. However, the present disclosure provides additional or alternative means that satisfy a long felt need in the art to regenerate injured discs and/or to facilitate inhibition of nucleus pulposus cell apoptosis.

### BRIEF SUMMARY

The present disclosure is directed to methods and compositions related to treatment or prevention of disc degeneration in a mammal. In particular embodiments, the present disclosure provides a concentrated conditioned media from de-differentiated fibroblasts that possesses the ability to regenerate injured discs and/or to facilitate inhibition of nucleus pulposus cell apoptosis. Some embodiments concern means for purification of exosomes from conditioned media from de-differentiated fibroblasts.

In one embodiment, there is a method of treating disc degeneration in an individual, comprising the step of providing to the individual an effective amount of conditioned media from culture of de-differentiated fibroblasts or one or more components therefrom. The conditioned media may comprise exosomes. In particular cases, the exosomes express markers selected from the group consisting of a) CD63; b) CD9; c) MHC I; d) CD56; and e) a combination thereof. The exosomes may or may not have been tested for expression of one or more markers prior to providing to the individual. In specific cases, the method further comprises a step of de-differentiating the fibroblasts to produce de-differentiated fibroblasts, for example to produce de-differentiated fibroblasts in culture. In specific embodiments, de-differentiated fibroblasts are produced upon exposure to stem cells and/or cytoplasm from stem cells. Examples of stem cells include at least pluripotent stem cells selected from the group consisting of a) parthenogenic stem cells; b) embryonic stem cells; c) inducible pluripotent stem cells; d) somatic cell nuclear transfer derived stem cells; e) Stimulus-triggered acquisition of pluripotency (STAP); and f) a combination thereof. In some cases, the de-differentiated fibroblasts are produced upon exposure to one or more particular conditions or compositions, such as hypoxia; one or more histone deacetylase inhibitors; one or more DNA methyltransferase inhibitors; and so forth. Examples of histone deacetylase inhibitors include those selected from the group consisting of a) valproic acid; b) trichostatin A; c) phenylbutyrate; d) vorinostat; e) belinostat; f) LAQ824; g) panobinostat; h) entinostat; i) CI994; j) mocetinostat; k) sulforaphane; and 1) a combination thereof. Examples of one or more DNA methyltransferase inhibitors include those selected from the group consisting of a) decitabine; b) 5-azacytidine; c) Zebularine; d) RG-108; e) procaine hydrochloride; f) Procainamide hydrochloride; g) Hydralazine hydrochloride; h) Epigallocatechin gallate; i) Chlorogenic acid; j) Caffeic acid; and h) a combination thereof.

In particular embodiments, the de-differentiated fibroblasts are produced upon exposure to 2%-8%, 2%-7%, 2%-6%, 2%-5%, 2%-4%, 2%-3%, 3%-8%, 3%-7%, 3%-6%, 3%-5%, 3%-4%, 4%-8%, 4%-7%, 4%-6%, 4%-5%, 5%-8%, 5%-7%, 5%-6%, 6%-8%, 6%-7%, or 7%-8% oxygen.

In particular embodiments, the exosomes are obtained from de-differentiated fibroblasts, and the exosomes may or may not be purified from culture of the de-differentiated fibroblasts using anion exchange chromatography, high performance liquid chromatography (HPLC), or both. In particular embodiments, the method comprises administering to the individual one or more of hyperbaric oxygen, adipose stem cell administration, bone marrow mesenchymal stem cell administration, fibroblast administration, and a combination thereof.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE FIGURES

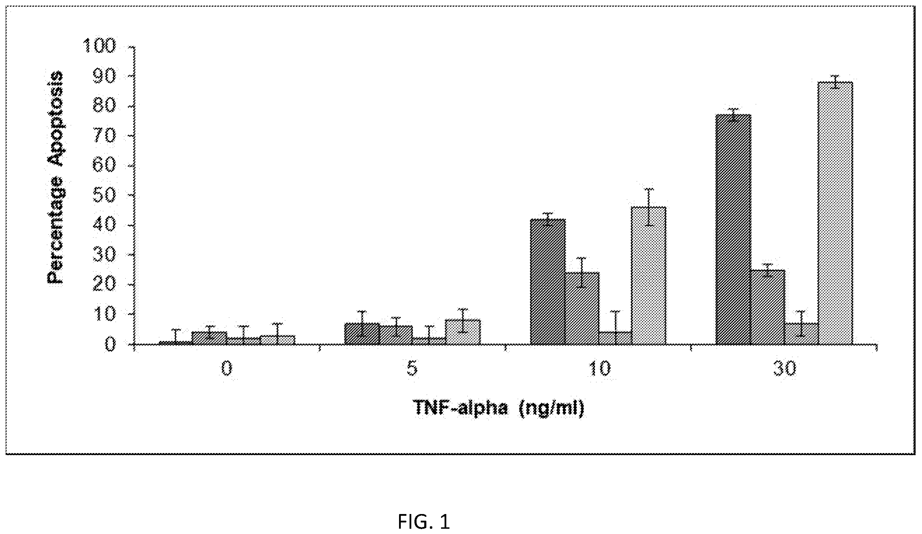

FIG. 1 shows that exosomes from fibroblasts inhibited TNF-alpha induced apoptosis, whereas exosomes from dedifferentiated fibroblasts even more potently inhibited suppressed apoptosis. Blue bars are saline control, orange bars are from fibroblasts, grey bars are from dedifferentiated fibroblasts (hypoxia and valproic acid) and dark yellow bars are fetal calf serum exosomes.

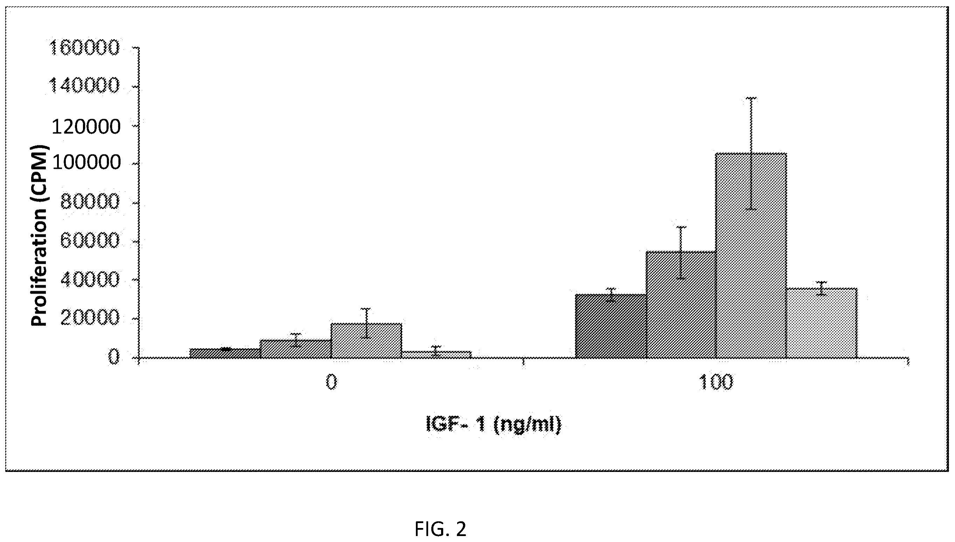

FIG. 2 shows that exosomes from fibroblasts inhibited stimulated NP cell proliferation, whereas exosomes from dedifferentiated fibroblasts even more potently stimulatory. Blue bars are saline control, orange bars are from fibroblasts, grey bars are from dedifferentiated fibroblasts (hypoxia and valproic acid) and dark yellow bars are fetal calf serum exosomes.

### DETAILED DESCRIPTION

### I. Examples of Definitions

In keeping with long-standing patent law convention, the words "a" and "an" when used in the present specification in concert with the word comprising, including the claims, denote "one or more." Some embodiments of the disclosure may consist of or consist essentially of one or more elements, method steps, and/or methods of the disclosure. It is contemplated that any method or composition described herein can be implemented with respect to any other method or composition described herein.

As used herein, the term "about" or "approximately" refers to a quantity, level, value, number, frequency, percentage, dimension, size, amount, weight or length that varies by as much as 30, 25, 20, 25, 10, 9, 8, 7, 6, 5, 4, 3, 2 or 1 % to a reference quantity, level, value, number, frequency, percentage, dimension, size, amount, weight or length. In particular embodiments, the terms "about" or "approximately" when preceding a numerical value indicates the value plus or minus a range of 15%, 10%, 5%, or 1%. With respect to biological systems or processes, the term can mean within an order of magnitude, preferably within 5-fold, and more preferably within 2-fold, of a value. Unless otherwise stated, the term 'about' means within an acceptable error range for the particular value.

The term "administered" or "administering", as used herein, refers to any method of providing a composition to an individual such that the composition has its intended effect on the patient. For example, one method of administering is by an indirect mechanism using a medical device such as, but not limited to a catheter, applicator gun, syringe *etc.* A second exemplary method of administering is by a direct mechanism such as, local tissue administration, oral ingestion, transdermal patch, topical, inhalation, suppository *etc.*

As used herein, "allogeneic" refers to tissues or cells or other material from another body that in a natural setting are immunologically incompatible or capable of being immunologically incompatible, although from one or more individuals of the same species.

As used herein, the term "allotransplantation" refers to the transplantation of organs, tissues, and/or cells from a donor to a recipient, where the donor and recipient are different individuals, but of the same species. Tissue transplanted by such procedures is referred to as an allograft or allotransplant.

As used herein, the terms "allostimulatory" and "alloreactive" refer to stimulation and reaction of the immune system in response to an allologous antigens, or "alloantigens" or cells expressing a dissimilar HLA haplotype.

As used herein, "autologous" refers to tissues or cells or other material that are derived or transferred from the same individual's body (*i.e.*, autologous blood donation; an autologous bone marrow transplant).

As used herein, the term "autotransplantation" refers to the transplantation of organs, tissues, and/or cells from one part of the body in an individual to another part in the same individual, *i.e.,* the donor and recipient are the same individual. Tissue transplanted by such "autologous" procedures is referred to as an autograft or autotransplant.

The term "biologically active" refers to any molecule having structural, regulatory or biochemical functions. For example, biological activity may be determined, for example, by restoration of wild-type growth in cells lacking protein activity. Cells lacking protein activity may be produced by many methods (*i.e.*, for example, point mutation and frame-shift mutation). Complementation is achieved by transfecting cells that lack protein activity with an expression vector that expresses the protein, a derivative thereof, or a portion thereof. In other cases, a fragment of a gene product (such as a protein) may be considered biologically active (or it may be referred to as functionally active) if it retains the activity of the full-length gene product, although it may be at a reduced but detectable level of the activity of the full-length gene product.

"Cell culture" is an artificial *in vitro* system containing viable cells, whether quiescent, senescent or (actively) dividing. In a cell culture, cells are grown and maintained at an appropriate temperature, typically a temperature of 37°C and under an atmosphere typically containing oxygen and CO₂, although in other cases these are altered. Culture conditions may vary widely for each cell type though, and variation of conditions for a particular cell type can result in different phenotypes being expressed. The most commonly varied factor in culture systems is the growth medium. Growth media can vary in concentration of nutrients, growth factors, and the presence of other components. The growth factors used to supplement media are often derived from animal blood, such as calf serum.

Throughout this specification, unless the context requires otherwise, the words "comprise", "comprises" and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that no other elements are optional and may or may not be present depending upon whether or not they affect the activity or action of the listed elements.

The term "drug", "agent" or "compound" as used herein, refers to any pharmacologically active substance capable of being administered that achieves a desired effect. Drugs or compounds can be synthetic or naturally occurring, non-peptide, proteins or peptides, oligonucleotides, or nucleotides (DNA and/or RNA), polysaccharides or sugars.

The term "individual", as used herein, refers to a human or animal that may or may not be housed in a medical facility and may be treated as an outpatient of a medical facility. The individual may be receiving one or more medical compositions via the internet. An individual may comprise any age of a human or non-human animal and therefore includes both adult and juveniles (*i.e.*, children) and infants. It is not intended that the term "individual" connote a need for medical treatment, therefore, an individual may voluntarily or involuntarily be part of experimentation whether clinical or in support of basic science studies. The term "subject" or "individual" refers to any organism or animal subject that is an object of a method or material, including mammals, *e.g.,* humans, laboratory animals (*e.g.*, primates, rats, mice, rabbits), livestock (*e.g.*, cows, sheep, goats, pigs, turkeys, and chickens), household pets (*e.g.*, dogs, cats, and rodents), horses, and transgenic non-human animals.

Reference throughout this specification to "one embodiment," "an embodiment," "a particular embodiment," "a related embodiment," "a certain embodiment," "an additional embodiment," or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments.

The term "pharmaceutically" or "pharmacologically acceptable", as used herein, refer to molecular entities and compositions that do not produce adverse, allergic, or other untoward reactions when administered to an animal or a human.

The term, "pharmaceutically acceptable carrier", as used herein, includes any and all solvents, or a dispersion medium including, but not limited to, water, ethanol, polyol (for example, glycerol, propylene glycol, and liquid polyethylene glycol, and the like), suitable mixtures thereof, and vegetable oils, coatings, isotonic and absorption delaying agents, liposome, commercially available cleansers, and the like. Supplementary bioactive ingredients also can be incorporated into such carriers.

The terms "reduce," "inhibit," "diminish," "suppress," "decrease," "prevent" and grammatical equivalents (including "lower," "smaller," *etc.*) when in reference to the expression of any symptom in an untreated subject relative to a treated subject, mean that the quantity and/or magnitude of the symptoms in the treated subject is lower than in the untreated subject by any amount that is recognized as clinically relevant by any medically trained personnel. In one embodiment, the quantity and/or magnitude of the symptoms in the treated subject is at least 10% lower than, at least 25% lower than, at least 50% lower than, at least 75% lower than, and/or at least 90% lower than the quantity and/or magnitude of the symptoms in the untreated subject.

"Therapeutic agent" means to have "therapeutic efficacy" in modulating angiogenesis and/or wound healing and an amount of the therapeutic is said to be a "angiogenic modulatory amount", if administration of that amount of the therapeutic is sufficient to cause a significant modulation (*i.e.*, increase or decrease) in angiogenic activity when administered to a subject (*e*.*g*., an animal model or human patient) needing modulation of angiogenesis.

As used herein, the term "therapeutically effective amount" is synonymous with "effective amount", "therapeutically effective dose", and/or "effective dose" and refers to the amount of compound that will elicit the biological, cosmetic or clinical response being sought by the practitioner in an individual in need thereof. As one example, an effective amount is the amount sufficient to reduce immunogenicity of a group of cells. As a non-limiting example, an effective amount is an amount sufficient to promote formation of a blood supply sufficient to support the transplanted tissue. As another non-limiting example, an effective amount is an amount sufficient to promote formation of new blood vessels and associated vasculature (angiogenesis) and/or an amount sufficient to promote repair or remodeling of existing blood vessels and associated vasculature. The appropriate effective amount to be administered for a particular application of the disclosed methods can be determined by those skilled in the art, using the guidance provided herein. For example, an effective amount can be extrapolated from *in vitro* and *in vivo* assays as described in the present specification. One skilled in the art will recognize that the condition of the individual can be monitored throughout the course of therapy and that the effective amount of a compound or composition disclosed herein that is administered can be adjusted accordingly.

As used herein, the term "transplantation" refers to the process of taking living tissue or cells and implanting it in another part of the body or into another body.

"Treatment," "treat," or "treating" means a method of reducing the effects of a disease or condition. Treatment can also refer to a method of reducing the disease or condition itself rather than just the symptoms. The treatment can be any reduction from pre-treatment levels and can be but is not limited to the complete ablation of the disease, condition, or the symptoms of the disease or condition. Therefore, in the disclosed methods, treatment" can refer to a 10%, 20%, 30%, 40%, 50%, 60%, 70%, 80%, 90%, or 100% reduction in the severity of an established disease or the disease progression, including reduction in the severity of at least one symptom of the disease. For example, a disclosed method for reducing the immunogenicity of cells is considered to be a treatment if there is a detectable reduction in the immunogenicity of cells when compared to pre-treatment levels in the same subject or control subjects. Thus, the reduction can be a 10, 20, 30, 40, 50, 60, 70, 80, 90, 100%, or any amount of reduction in between as compared to native or control levels. It is understood and herein contemplated that "treatment" does not necessarily refer to a cure of the disease or condition, but an improvement in the outlook of a disease or condition. In specific embodiments, treatment refers to the lessening in severity or extent of at least one symptom and may alternatively or in addition refer to a delay in the onset of at least one symptom.

### II. Media from De-Differentiated Fibroblasts and Components Thereof

This disclosure relates to methods and compositions for treating or preventing pathological intervertebral discs by delivering one or more compositions produced from, such as secreted by, fibroblasts in which the fibroblasts have been de-differentiated and optionally stimulated *in vitro.* Methods are provided in accordance with the current disclosure concerning means of obtaining a conditioned media possessing one or more agents suitable for administration into an intervertebral disc, including multifactorially-acting agents suitable for administration into an intervertebral disc, including in a manner to stimulate regeneration of the disc or disc component(s). One embodiment encompasses de-differentiated fibroblasts that are re-differentiated into nucleus pulposus (NP) cells, or NP-like cells, and the cells are subsequently exposed to one or more stressors, allowing for release of one or more regenerative factors. One or more of these factors, in specific cases in a medium, are provided to an individual in need thereof.

Embodiments of the disclosure encompass particular conditioned media, including for therapeutic use. In specific embodiments, the conditioned media is useful for stimulation of disc regeneration in an individual, including one suffering from disc degenerative disease or at risk for disc degenerative disease (an individual at risk is an individual over the age of about 40, 45, 50, 55, 60, 65, 70, 75, 80, and so forth; an individual that is or was an athlete; an individual with a vocation that requires physical activity; an individual with a spinal injury; or a combination thereof, for example). Thus, in particular embodiments disc degeneration is prevented utilizing methods encompassed by the disclosure or the disc degeneration may be delayed in onset and/or reduced in severity.

In particular embodiments, the conditioned media may be generated by manipulation of fibroblast cells that may be any kind of fibroblast cells. Such manipulation of fibroblasts includes, in some embodiments, (a) exposing fibroblast cells to culture conditions and/or one or more agents capable of inducing de-differentiation of the fibroblasts; (b) stimulating re-differentiation of the fibroblasts towards a phenotype resembling nucleus pulposus (NP) cells; c) exposing the re-differentiated NP cells to one or more stressor conditions; d) extracting conditioned media generated by the re-differentiated NP cells in response to their exposure to the stressor condition(s); and (e) administering the conditioned media into an individual in need of therapy.

In some cases, fibroblast cells are obtained from a biopsy, and the donor providing the biopsy may be either the individual to be treated (autologous), or the donor may be different from the individual to be treated (allogeneic). In cases wherein allogeneic fibroblast cells are utilized for an individual, the fibroblast cells may come from one or a plurality of donors.

The fibroblasts may be obtained from a source selected from the group consisting of: a) dermal fibroblasts; b) placental fibroblasts; c) adipose fibroblasts; d) bone marrow fibroblasts; e) foreskin fibroblasts; f) umbilical cord fibroblasts; g) hair follicle derived fibroblasts; h) nail derived fibroblasts; i) endometrial derived fibroblasts; j) keloid derived fibroblasts; and k) a combination thereof.

In particular embodiments fibroblasts are induced to de-differentiate, and the de-differentiated cells are manipulated to produce certain factor(s). In specific embodiments, induction of de-differentiation of the fibroblasts is performed by culture of the fibroblasts together with cytoplasm from a cell possessing a more undifferentiated phenotype, as compared to original fibroblasts. In some cases, de-differentiation of the fibroblasts is performed by culture of the fibroblasts with cells possessing a more undifferentiated phenotype. In specific cases, cells possessing a more undifferentiated phenotype may be any kind of stem cell, for example, such as pluripotent stem cells, including pluripotent stem cells selected from the group of cells consisting of a) parthenogenic stem cells; b) embryonic stem cells; c) inducible pluripotent stem cells; d) somatic cell nuclear transfer derived stem cells; e) Stimulus-triggered acquisition of pluripotency (STAP); and f) a combination thereof. In particular embodiments fibroblasts are induced to de-differentiate using one or more de-differentiation agents that comprise cytoplasm(s) derived from stem cells, including pluripotent stem cells. In specific cases, cytoplasm derived from stem cells, including pluripotent stem cells is transfected into fibroblasts. The cytoplasm-transfected de-differentiated fibroblasts may be cultured and the subsequent media is provided in sufficient amounts to an individual, such as at one or more discs in the individual.

In certain embodiments, de-differentiated fibroblasts are produced upon culture under hypoxia. In particular embodiments, culture of fibroblasts with undifferentiated cells (and/or cytoplasm from undifferentiated cells) is performed under conditions of hypoxia, such as culture in conditions of reduced oxygen as compared to atmospheric oxygen. In specific cases, reduced oxygen is between 0.2%-5% oxygen, 0.2%-4%, 0.2%-3%, 0.2%-2%, 0.2%-1%, 0.2%-0.75%, 0.2%-0.5%, 0.5%-5%, 0.5%-4%, 0.5%-3%, 0.5%-2%, 0.5%-1%, 0.5%-0.75%, 0.75%-5%, 0.75%-4%, 0.75%-3%, 0.75%-2%, 0.75%-1%, 1%-5%, 1%-4%, 1%-3%, 1%-2%, 2%-5%, 2%-4%, 2%-3%, 3%-5%, 3%-4%, or 4%-5%% oxygen, in specific embodiments. The duration of exposure of the cells to hypoxic conditions, including with (but not limited to) these representative levels of oxygen, may be for a duration of 30 minutes (min)-3 days, 30 min-2 days, 30 min-1 day, 30 min-12 hours (hrs), 30 min-8 hrs, 30 min-6 hrs, 30 min-4 hrs, 30 min-2 hrs, 30 min-1 hour (hr), 1 hr-3 days, 1hr-2 days, 1 hr-1 day, 1-12 hrs, 1-8 hrs, 1-6 hrs, 1-4 hrs, 1-2 hrs, 2 hrs-3 days, 2hrs-2 days, 2 hrs-1 day, 2 hrs-12 hrs, 2-10hrs, 2-8hrs, 2-6 hrs, 2-4 hrs, 2-3 hrs, 6 hrs-3 days, 6 hrs-2 days, 6 hrs-1 day, 6-12 hrs, 6-8 hrs, 8hrs-3 days, 8 hrs-2 days, 8 hrs-1 day, 8-16 hrs, 8-12 hrs, 8-10 hrs, 12hrs-3 days, 12 hrs-2 days, 12 hrs-1 day, 12-18hrs, 12-14hrs, 1-3 days, or 1-2 days, as examples only.

In particular embodiments, culture of fibroblasts with undifferentiated cells (and/or cytoplasm from undifferentiated cells) is performed under conditions including the presence of one or more histone deacetylase inhibitors, such as a histone deacetylase inhibitor selected from a group consisting of: a) valproic acid; b) trichostatin A; c) phenylbutyrate; d) vorinostat; e) belinostat; f) LAQ824; g) panobinostat; h) entinostat; i) CI994; j) mocetinostat; k) sulforaphane; and l) a combination thereof. In specific cases exposure of the undifferentiated cells (and/or cytoplasm from undifferentiated cells) with one or more histone deacetylase inhibitors enhances the ability of the de-differentiated fibroblasts to cause regeneration of one or more discs of an individual.

In particular embodiments, culture of fibroblasts with undifferentiated cells (and/or cytoplasm from undifferentiated cells) is performed under conditions including the presence of one or more DNA methyltransferase inhibitors. The DNA methyltransferase inhibitor may be selected from the group consisting of: a) decitabine; b) 5-azacytidine; c) Zebularine; d) RG-108; e) procaine hydrochloride; f) Procainamide hydrochloride; g) Hydralazine hydrochloride; h) Epigallocatechin gallate; i) Chlorogenic acid; j) Caffeic acid; and h) a combination thereof. In specific cases exposure of the undifferentiated cells (and/or cytoplasm from undifferentiated cells) with one or more DNA methyltransferase inhibitors enhances the ability of the de-differentiated fibroblasts to cause regeneration of one or more discs of an individual.

In particular cases, media allowing for de-differentiated fibroblast proliferation comprises one or more factors known to be mitogenic for dedifferentiated fibroblasts, such as one or more factors selected from the group consisting of: a) FGF-1; b) FGF-2; c) FGF-5; d) EGF; e) CNTF; f) KGF-1; g) PDGF; h) platelet rich plasma; i) TGF-alpha; j) HGF-1; and k) a combination thereof. In specific cases, exposure of the undifferentiated cells (and/or cytoplasm from undifferentiated cells) with one or more factors known to be mitogenic for dedifferentiated fibroblasts (for example, in culture) enhances the ability of the de-differentiated fibroblasts to cause regeneration of one or more discs of an individual.

In specific embodiments, fibroblasts subsequent to de-differentiation are cultured to obtained a conditioned media. In certain cases, the fibroblasts subsequent to de-differentiation are cultured that results in the production of exosomes from the de-differentiated cells, and exosomes are obtained from the conditioned media. In particular cases, the exosomes are collected from de-differentiated fibroblasts while the fibroblasts are in a proliferating state. Exosomes may be collected from de-differentiated fibroblasts while the de-differentiated fibroblasts are cultured in a media comprising no proliferative factors or largely reduced levels of proliferation-inducing growth factors. Exosomes may be collected from de-differentiated fibroblasts that have been cultured in media with certain levels of oxygen for a certain duration of time. For example, exosomes may be collected from de-differentiated fibroblasts that have been cultured in media with 2%-8%, 2%-7%, 2%-6%, 2%-5%, 2%-4%, 2%-3%, 3%-8%, 3%-7%, 3%-6%, 3%-5%, 3%-4%, 4%-8%, 4%-7%, 4%-6%, 4%-5%, 5%-8%, 5%-7%, 5%-6%, 6%-8%, 6%-7%, or 7%-8% oxygen, as examples. Exosomes may be collected from de-differentiated fibroblasts that have been cultured in media for a certain duration of time, and this duration may or may not include the above noted levels of oxygen. Exosomes may be collected from de-differentiated fibroblasts that have been cultured in media for at least 1, 2, 3, 4, 5, 6, 7, 8, 9, 10, 11, 12, 13, 14, or 15 days. The cells may be cultured for 1-15, 1-14, 1-13, 1-12, 1-11, 1-10, 1-9, 1-8, 1-7, 1-6, 1-5, 1-4, 1-3, 1-2, 2-15, 2-14, 2-13, 2-12, 2-11, 2-10, 2-9, 2-8, 2-7, 2-6, 2-5, 2-4, 2-3, 3-15, 3-14, 3-13, 3-12, 3-11, 3-10, 3-9, 3-8, 3-7, 3-6, 3-5, 3-4, 4-15, 4-14, 4-13, 4-12, 4-11, 4-10, 4-9, 4-8, 4-7, 4-6, 4-5, 5-15, 5-14, 5-13, 5-12, 5-11, 5-10, 5-9, 5-8, 5-7, 5-6, 6-15, 6-14, 6-13, 6-12, 6-11, 6-10, 6-9, 6-8, 6-7, 7-15, 7-14, 7-13, 7-12, 7-11, 7-10, 7-9, 7-8, 8-15, 8-14, 8-13, 8-12, 8-11, 8-10, 8-9, 9-15, 9-14, 9-13, 9-12, 9-11, 9-10, 10-15, 10-14, 10-13, 10-12, 10-11, 11-15, 11-14, 11-13, 11-12, 12-15, 12-14, 12-13, 13-15, 13-14, or 14-15 days, for example.

In some cases the de-differentiated fibroblasts (or any cells in any method encompassed herein) are passaged, for example for at least 1, 2, 3, 4, 5, 6, 7, 8, 9, 10, 12, 13, 14, 15, 16, 17, 18, 19, 20, or more passages.

Exosomes in the context of the present disclosure may be present in a preparation. In specific cases the exosomes are in a preparation that comprises less than 5%, 4%, 3%, 2%, or 1% polyethylene glycol. The exosomes may be purified using polyethylene glycol, using ultrafiltration, or both, for example. In specific embodiments, polyethylene glycol is added to the exosomes after purification. Exosomes may express markers selected from the group consisting of: a) CD63; b) CD9; c) MHC I; d) CD56; and e) a combination thereof.

De-differentiated fibroblasts may be cultured in any media, but in particular cases the media is selected from the group consisting of: a) Roswell Park Memorial Institute (RPMI-1640); b) Dublecco's Modified Essential Media (DMEM), c) Eagle's Modified Essential Media (EMEM), d) Optimem, e) Iscove's Media, and f) a combination thereof.

The disclosure encompasses means of treating degenerative disc disease in an individual by administration to the individual of an effective amount of conditioned media from de-differentiated fibroblasts, and/or conditioned media from de-differentiated fibroblasts that have subsequently been re-differentiated into notochord or NP cells. The media may or may not further comprise de-differentiated fibroblasts.

For the purpose of the disclosure, fibroblasts may be treated with a variety of de-differentiated compositions that can endow increased pluripotency. In one aspect of the disclosure, fibroblasts are treated with cytoplasm from a more undifferentiated cell. Such cells, such as pluripotent stem cells, are well known in the art, and methods of derivation are also well known. Without limitation, useful pluripotent cells of extraction of cytoplasm include parthenogenic stem cells [24-38], embryonic stem cells [39, 40], inducible pluripotent stem cells [41-45], stimulus-triggered acquisition of pluripotency (STAP) [46], and somatic cell nuclear transfer derived stem cells [47-49].

Extraction of cytoplasmic matter may be performed as described in the art. In one embodiment, pluripotent cells are made to enter the interphase stage of cell cycle and are harvested using standard methods and washed by centrifugation at 500xg for 10 minutes in a 10 ml conical tube at 4° C. The supernatant is discarded, and the cell pellet is re-suspended in a total volume of 50 ml of cold PBS. The cells are centrifuged at 500xg for 10 minutes at 4° C. This washing step is repeated, and the cell pellet is resuspended in approximately 20 volumes of ice-cold interphase cell lysis buffer (20 mM Hepes, pH 8.2, 5 mM MgCl₂, 1 mM DTT, 10 .mu.M aprotinin, 10 .mu.M leupeptin, 10 .mu.M pepstatin A, 10 µM soybean trypsin inhibitor, 100 µM PMSF, and preferably 20 µg/ml cytochalasin B). The cells are sedimented by centrifugation at 800xg for 10 minutes at 4°C. The supernatant is discarded, and the cell pellet is carefully resuspended in no more than one volume of interphase cell lysis buffer. The cells are incubated on ice for one hour to allow swelling of the cells. The cells are lysed by either sonication using a tip sonicator or Dounce homogenization using a glass mortar and pestle. Cell lysis is performed until at least 90% of the cells and nuclei are lysed, which may be assessed using phase contrast microscopy. The sonication time required to lyse at least 90% of the cells and nuclei may vary depending on the type of cell used to prepare the extract. The cell lysate is placed in a 1.5-ml centrifuge tube and centrifuged at 10,000 to 15,000xg for 15 minutes at 4°C. using a table top centrifuge. The tubes are removed from the centrifuge and immediately placed on ice. The supernatant is carefully collected using a 200 µl pipette tip, and the supernatant from several tubes is pooled and placed on ice. This supernatant is the "interphase cytoplasmic" or "IS15" extract. This cell extract may be aliquoted into 20 µl volumes of extract per tube on ice and immediately flash-frozen on liquid nitrogen and stored at -80°C. until use. Alternatively, the cell extract is placed in an ultracentrifuge tube on ice (*e.g.*, fitted for an SW55 Ti rotor; Beckman). If necessary, the tube is overlayed with mineral oil to the top. The extract is centrifuged at 200,000xg for three hours at 4°C to sediment membrane vesicles contained in the IS15 extract. At the end of centrifugation, the oil is discarded. The supernatant is carefully collected, pooled if necessary, and placed in a cold 1.5 ml tube on ice. This supernatant is referred to as "IS200" or "interphase cytosolic" extract. The extract is aliquoted and frozen as described for the IS15 extract. If desired, the extract can be enriched with additional nuclear factors. For example, nuclei can be purified from cells of the cell type from which the reprogramming extract is derived and lysed by sonication as described above. The nuclear factors are extracted by a 10-60 minute incubation in nuclear buffer containing NaCl or KCl at a concentration of 0.15-800 mM under agitation. The lysate is centrifuged to sediment unextractable components. The supernatant containing the extracted factors of interest is dialyzed to eliminate the NaCl or KCl. The dialyzed nuclear extract is aliquoted and stored frozen. This nuclear extract is added at various concentrations to the whole cell extract described above prior to adding the nuclei for reprogramming. As an alternative to a cell extract, a reprogramming media can also be formed by adding one or more naturally-occurring or recombinant factors (e.g., nucleic acids or proteins such as T-cell receptors or other signaling surface molecules, DNA methyltransferases, histone deacetylases, histones, nuclear lamins, transcription factors, activators, repressors, growth factors, hormones, or cytokines) to a solution, such as a buffer. Preferably, one or more of the factors are specific for the cell type one wishes the donor cell to become.

The extract can be used for reprogramming of fibroblasts by culture. In one embodiment, fibroblasts grown on coverslips are reversibly permeabilized with the bacterial toxin Streptolysin O, exposed to extracts of pluripotent stem cells and resealed with 2 mM CaCl₂, and expanded in culture. In one embodiment, fibroblasts are grown on 16-mm poly-L-lysine-coated coverslips in RPMI1640 to 100,000 cells/coverslip in 12-well plates. Cells are permeabilized in 200 ng/ml streptolysin O in Ca²⁺-free Hanks Balanced Salt Solution (Gibco-BRL) for 50 minutes at 37°C. in regular atmosphere. Over 80% of fibroblasts cells are permeabilized under these conditions, as judged by propidium iodide uptake. Streptolysin O is aspirated; coverslips overlaid with 80 µl of either pluripotent stem cell extract; and incubated for one hour at 37°C. in CO₂ atmosphere. Each extract contained the ATP generating system and 1 mM each of ATP, CTP, GTP and UTP. Extracts from pluripotent stem cells are prepared as described above. To reseal plasma membranes, RPMI1640 containing 2 mM CaCl₂ (added from a 1 M stock in H₂O) is added to the wells, and the cells are incubated for two hours at 37°C. This procedure resealed about 100% of the permeabilized cells. Ca²⁺-containing RPMI was replaced by RPMI, and the cells are expanded for several weeks.

Several descriptions of cytoplasmic transferring have been published and are incorporated by reference [50-52]. Once de-differentiated fibroblasts are obtained, conditioned media, and/or exosomes from the conditioned media, are administered to an individual in need thereof. The conditioned media, and/or exosomes from the conditioned media, may be concentrated and used therapeutically, for example by intradiscal administration.

In some embodiments, de-differentiated fibroblasts are re-differentiated into notochord cells and conditioned media is extracted from notochord cells for use directly or as a therapeutic source of exosomes.

In one embodiment, notochord cells are generated from de-differentiated fibroblasts by dissociation into single cells by incubating with Accutase (Millipore, Billerica, MA) for ~10 min at 37°C and gently pipetting. The dissociated cells are spun down and re-suspended in the fresh maintenance medium supplemented with 10 µM Y27632 for plating. Approximately 150,000 cells suspending in 2 ml medium are plated in each well of 6-well culture plates. Pulverized nucleus pulposus tissue is added at a suitable amount. The nucleus pulposus tissue is added either directly into each well (contact culture mode), or placed in an insert (70 um cell strainer; BD Bioscience) which is press-fitted in the culture wells; sufficient culture medium was added to the wells to cover the tissue in the insert (non-contact culture mode). After 24 hours, the culture medium is supplemented with an equal volume of differentiation medium. Two media may be used tested: Medium 1 contained alpha-minimum essential medium (α-MEM), 10% fetal bovine serum (FBS), 100 U/ml penicillin and 100 µg/ml streptomycin (all from Life Technologies, NY), while Medium 2 is commercially available EGM-2-MV BulletKit (Lonza, Walkersville, MD) that contained 5% FBS and supplements of a cocktail of growth factors (FGF, EGF, VEGF and IGF-1). Medium 2 is routinely used to derive mesodermal lineage cells. Since the notochord has a mesoderm origin, this Medium 2 promotes notochordal differentiation. The two-step procedure of changing medium is helpful to promote cell survival. Then, after two days, the medium is changed completely to the differentiation medium and replenished every two or three days thereafter. When changing the medium, care is taken to avoid to withdraw the NP tissue out of the medium. Other means of inducing dedifferentiated fibroblasts into notochord differentiation are known and are incorporated by reference [53-55].

In particular embodiments, media is at least part of a therapeutic product of the disclosure. In one embodiment, notochord and/or notochord-like cells are used as a source of conditioned media and/or exosomes. In some embodiments, the disclosure provides means of stimulating regeneration of degenerated discs using exosomes derived from de-differentiated fibroblast tissue cultures. In one embodiment, the disclosure encompasses the extraction of exosomes from cultures of de-differentiated fibroblasts, concentration of the exosomes, and administration of the exosomes for the purpose of stimulating regeneration of the discs; in some embodiments, administration of the exosomes additionally or alternatively provides protection from apoptosis and/or stimulation of endogenous chondrocytes. In other embodiments, a combination of exosomes from de-differentiated fibroblasts is provided to the individual with regenerative exogenous stem cells. Without being restricted to mechanism, exosomes produced by tissue culture may stimulate regeneration through directly acting as mitogens for chondrocytes/notochord cells and/or may stimulate regeneration by inducing production of pro-regenerative cytokines in cells of the disc, for example; in another embodiment, stimulation of proteoglycan production is achieved.

In one embodiment, de-differentiated fibroblasts are cultured using means known in the art for preserving viability and proliferative ability of cells such as pluripotent cells, or fibroblasts. The disclosed methods and compositions may be applied both for individualized autologous exosome preparations and for exosome preparations obtained from established cell lines, for experimental or biological use, and/or from one or more donors.

### III. Preparation of Disc Regenerative Membrane Vesicles

In one embodiment, this disclosure encompasses the use of chromatography separation methods (as one example) for preparing disc regenerative membrane vesicles, particularly to separate the membrane vesicles from potential biological contaminants, wherein the microvesicles are exosomes, and cells utilized for generating the exosomes are de-differentiated fibroblast cells.

Membrane vesicles, including exosomes, could be purified, and possess ability to stimulate angiogenesis in at least some cases. In one embodiment, a strong or weak anion exchange may be performed as part of the purification. In addition, in a specific embodiment, the chromatography is performed under pressure. Thus, more specifically, it may comprise high performance liquid chromatography (HPLC). Different types of supports may be used to perform the anion exchange chromatography. In particular, these may include cellulose, poly(styrenedivinylbenzene), agarose, dextran, acrylamide, silica, ethylene glycol-methacrylate co-polymer, or mixtures thereof, *e*.*g*., agarose-dextran mixtures. To illustrate this, one can utilize the different chromatography equipment comprised of supports as mentioned above, particularly the following gels: POROS^{®}, SEPHAROSE^{®}, SEPHADEX^{®}, TRISACRYL^{®}, TSK-GEL SW or PW^{®}, SUPERDEX^{®}TOYOPEARL HW and SEPHACRYL^{®}, for example, which are suitable for use with methods of the disclosure. Therefore, in a specific embodiment, this disclosure relates to a method of preparing membrane vesicles, particularly exosomes, from a biological sample such as a tissue culture comprising de-differentiated fibroblasts, comprising at least one step during which the biological sample is treated by anion exchange chromatography on a support selected from cellulose, poly(styrene-divinylbenzene), silica, acrylamide, agarose, dextran, and ethylene glycol-methacrylate co-polymer, alone or in mixtures, optionally functionalized.

In some embodiments, one can utilize supports in bead form. In at least some cases, these beads have a homogeneous and calibrated diameter, with a sufficiently high porosity to enable the penetration of the objects under chromatography (*i.e.*, the exosomes). In this way, given the diameter of exosomes (generally between 50 and 100 nm), to apply to methods encompassed herein, one can use high porosity gels, particularly between 10 nm and 5 µm, including between approximately 20 nm and approximately 2 µm, including between about 100 nm and about 1 µm. For the anion exchange chromatography, the support used must be functionalized using a group capable of interacting with an anionic molecule. Generally, this group is composed of an amine that may be ternary or quaternary, which defines a weak or strong anion exchanger, respectively. Within the scope of this disclosure, one can use a strong anion exchanger. In this way, according to the disclosure, a chromatography support as described above, functionalized with quaternary amines, is used. Therefore, according to a more specific embodiment of the disclosure, the anion exchange chromatography is performed on a support functionalized with a quaternary amine. In certain cases, this support should be selected from poly(styrene-divinylbenzene), acrylamide, agarose, dextran and silica, alone or in mixtures, and functionalized with a quaternary amine. Examples of supports functionalized with a quaternary amine include the gels SOURCEQ. MONO Q, Q SEPHAROSE^{®}, POROS^{®} HQ and POROS^{®} QE, FRACTOGEL^{®}TMAE type gels and TOYOPEARL SUPER^{®}Q gels.

In a certain embodiment, one can perform the anion exchange chromatography that comprises poly(styrene-divinylbenzene). An example of this type of gel that may be used is SOURCE Q gel, including SOURCE 15 Q (Pharmacia). This support offers the advantage of very large internal pores, thus offering low resistance to the circulation of liquid through the gel, while enabling rapid diffusion of the exosomes to the functional groups, which are particularly important parameters for exosomes given their size. The biological compounds retained on the column may be eluted in different ways, particularly using the passage of a saline solution gradient of increasing concentration, *e.g*. from 0 to 2 M. A sodium chloride solution may particularly be used, in concentrations varying from 0 to 2 M, for example. The different fractions purified in this way are detected by measuring their optical density (OD) at the column outlet using a continuous spectro-photometric reading. As an indication, under the conditions used in the examples, the fractions comprising the membrane vesicles were eluted at an ionic strength comprised between approximately 350 and 700 mM, depending on the type of vesicles.

Different types of columns may be used to perform this chromatographic step, according to requirements and the volumes to be treated. For example, depending on the preparations, it is possible to use a column from approximately 100 µl up to 10 ml or greater. In this way, the supports available have a capacity that may reach 25 mg of proteins/ml, for example. For this reason, a 100 µl column has a capacity of approximately 2.5 mg of proteins which, given the samples in question, allows the treatment of culture supernatants of approximately 2 1 (which, after concentration by a factor of 10 to 20, for example, represent volumes of 100 to 200 ml per preparation). It is understood that higher volumes may also be treated, by increasing the volume of the column, for example. In addition, to perform this invention, it is also possible to combine the anion exchange chromatography step with a gel permeation chromatography step. In this way, according to a specific embodiment of the disclosure, a gel permeation chromatography step is added to the anion exchange step, either before or after the anion exchange chromatography step. In this embodiment, the permeation chromatography step takes place after the anion exchange step. In addition, in a specific variant, the anion exchange chromatography step is replaced by the gel permeation chromatography step. The present application demonstrates that membrane vesicles may also be purified using gel permeation liquid chromatography, particularly when this step is combined with an anion exchange chromatography or other treatment steps of the biological sample, as described in detail below.

To perform the gel permeation chromatography step, a support selected from silica, acrylamide, agarose, dextran, ethylene glycol-methacrylate co-polymer or mixtures thereof, *e*.*g*., agarose-dextran mixtures, may be used. As an illustration, for gel permeation chromatography, a support such as SUPERDEX^{®}200HR (Pharmacia), TSK G6000 (TosoHaas) or SEPHACRYL^{®} S (Pharmacia) may be used. The process according to the disclosure may be applied to different biological samples. In particular, these may comprise a biological fluid from a subject (bone marrow, peripheral blood, *etc*.), a culture supernatant, a cell lysate, a pre-purified solution or any other composition comprising membrane vesicles.

In this respect, in a specific embodiment of the disclosure, the biological sample is a culture supernatant of membrane vesicle-producing dedifferentiated fibroblast cells.

In addition, according to a particular embodiment of the disclosure, the biological sample is treated, prior to the chromatography step, to be enriched with membrane vesicles (enrichment stage). In this way, in a specific embodiment, this disclosure relates to a method of preparing membrane vesicles from a biological sample, characterized in that it comprises at least: b) an enrichment step, to prepare a sample enriched with membrane vesicles, and c) a step during which the sample is treated by anion exchange chromatography and/or gel permeation chromatography.

In one embodiment, the biological sample is a culture supernatant treated so as to be enriched with membrane vesicles. In particular, the biological sample may comprise a pre-purified solution obtained from a culture supernatant of a population of membrane vesicle-producing cells or from a biological fluid, by treatments such as centrifugation, clarification, ultrafiltration, nanofiltration and/or affinity chromatography, particularly with clarification and/or ultrafiltration and/or affinity chromatography. Therefore, a particular method of preparing membrane vesicles according to the disclosure comprises the following steps: a) culturing a population of membrane vesicle (*e*.*g*. exosome) producing cells under conditions enabling the release of vesicles, b) a step of enrichment of the sample in membrane vesicles, and c) an anion exchange chromatography and/or gel permeation chromatography treatment of the sample.

As indicated above, the sample (*e*.*g*., supernatant) enrichment step may comprise one or more of centrifugation, clarification, ultrafiltration, nanofiltration and/or affinity chromatography steps on the supernatant. In a first specific embodiment, the enrichment step comprises (i) the elimination of cells and/or cell debris (clarification), possibly followed by (ii) a concentration and/or affinity chromatography step. In another specific embodiment, the enrichment step comprises an affinity chromatography step, optionally preceded by a step of elimination of cells and/or cell debris (clarification). A particular enrichment step according to this disclosure comprises (i) the elimination of cells and/or cell debris (clarification), (ii) a concentration and (iii) an affinity chromatography. The cells and/or cell debris may be eliminated by centrifugation of the sample, for example, at a low speed, such as below 1000 g, between 100 and 700 g, for example. Preferred centrifugation conditions during this step are approximately 300 g or 600 g for a period between 1 and 15 minutes, for example.

The cells and/or cell debris may also be eliminated by filtration of the sample, possibly combined with the centrifugation described above. The filtration may particularly be performed with successive filtrations using filters with a decreasing porosity. For this purpose, filters with a porosity above 0.2 µm, *e*.*g*. between 0.2 and 10 µm, are preferentially used. It is particularly possible to use a succession of filters with a porosity of 10 µm, 1 µm, 0.5 µm followed by 0.22 µm.

A concentration step may also be performed, in order to reduce the volumes of sample to be treated during the chromatography stages. In this way, the concentration may be obtained by centrifugation of the sample at high speeds, *e*.*g*. between 10,000 and 100,000 g, to cause the sedimentation of the membrane vesicles. This may consist of a series of differential centrifugations, with the last centrifugation performed at approximately 70,000 g. The membrane vesicles in the pellet obtained may be taken up with a smaller volume and in a suitable buffer for the subsequent steps of the process. The concentration step may also be performed by ultrafiltration. In fact, this ultrafiltration allows one both to concentrate the supernatant and perform an initial purification of the vesicles. According to a particular embodiment, the biological sample (*e.g.*, the supernatant) is subjected to an ultrafiltration, such as a tangential ultrafiltration. Tangential ultrafiltration comprises concentrating and fractionating a solution between two compartments (filtrate and retentate), separated by membranes of determined cut-off thresholds. The separation is carried out by applying a flow in the retentate compartment and a transmembrane pressure between this compartment and the filtrate compartment. Different systems may be used to perform the ultrafiltration, such as spiral membranes (Millipore, Amicon), flat membranes or hollow fibers (Amicon, Millipore, Sartorius, Pall, GF, Sepracor). Within the scope of the disclosure, the use of membranes with a cut-off threshold below 1000 kDa, such as between 300 kDa and 1000 kDa, or even between 300 kDa and 500 kDa, may be utilized.

The affinity chromatography step can be performed in various ways, using different chromatographic support and material. It is advantageously a non-specific affinity chromatography, aimed at retaining (*i*.*e*., binding) certain contaminants present within the solution, without retaining the objects of interest (i.e., the exosomes). It is therefore a negative selection. In particular cases, an affinity chromatography on a dye is used, allowing the elimination (*i.e.*, the retention) of contaminants such as proteins and enzymes, for instance albumin, kinases, deshydrogenases, clotting factors, interferons, lipoproteins, or also co-factors, *etc.* In specific cases, the support used for this chromatography step is a support as used for the ion exchange chromatography, functionalized with a dye. As specific example, the dye may be selected from Blue SEPHAROSE^{®}(Pharmacia), YELLOW 86, GREEN 5 and BROWN 10 (Sigma). The support is more preferably agarose. It should be understood that any other support and/or dye or reactive group allowing the retention (binding) of contaminants from the treated biological sample can be used in the instant invention.

In one embodiment, there is a membrane vesicle preparation process within the scope of this disclosure that comprises the following steps: a) the culture of a population of membrane vesicle (*e*.*g*. exosome) producing cells (for example, fibroblasts and/or de-differentiated fibroblasts) under conditions enabling the release of vesicles, b) the treatment of the culture supernatant with at least one ultrafiltration or affinity chromatography step, to produce a biological sample enriched with membrane vesicles (*e*.*g*. with exosomes), and c) an anion exchange chromatography and/or gel permeation chromatography treatment of the biological sample. In a particular embodiment, step b) above comprises a filtration of the culture supernatant, followed by an ultrafiltration, preferably tangential. In another preferred embodiment, step b) above comprises a clarification of the culture supernatant, followed by an affinity chromatography on dye, preferably on Blue SEPHAROSE^{®}.

In addition, after step c), the material harvested may, if applicable, be subjected to one or more additional treatment and/or filtration stages d), particularly for sterilization purposes. For this filtration treatment stage, filters with a diameter less than or equal to 0.3 µm are preferentially used, or even more preferentially, less than or equal to 0.25 µm. Such filters have a diameter of 0.22 µm, for example.

After step d), the material obtained is, for example, distributed into suitable devices such as bottles, tubes, bags, syringes, *etc.,* in a suitable storage medium. The purified vesicles obtained in this way may be stored cold, frozen or used extemporaneously. Therefore, a specific preparation process within the scope of the disclosure comprises at least the following steps: c) an anion exchange chromatography and/or gel permeation chromatography treatment of the biological sample, and d) a filtration step, particularly sterilizing filtration, of the material harvested after stage c). In a first variant, the process according to the disclosure comprises: c) an anion exchange chromatography treatment of the biological sample, and d) a filtration step, particularly sterilizing filtration, on the material harvested after step c).

In another variant, the process according to the disclosure comprises: c) a gel permeation chromatography treatment of the biological sample, and d) a filtration step, particularly sterilizing filtration, on the material harvested after step c). According to a third variant, the process according to the disclosure comprises: c) an anionic exchange treatment of the biological sample followed or preceded by gel permeation chromatography, and d) a filtration step, particularly sterilizing filtration, on the material harvested after step c).

Further embodiments include a method of optimizing regeneration stimulating therapeutic factor production from de-differentiated fibroblast cultures through the use of filters that separate compositions based on electrical charge, size or ability to elute from an adsorbent. Numerous techniques are known in the art for purification of therapeutic factors and concentration of agents. For some particular uses the de-differentiated fibroblast derived compounds will be sufficient for use as culture supernatants of the cells in media. Currently media useful for this purpose include Roswell Park Memorial Institute (RPMI-1640), Dublecco's Modified Essential Media (DMEM), Eagle's Modified Essential Media (EMEM), Optimem, and Iscove's Media.

In one embodiment, therapeutic factors for stimulating regeneration are derived from tissue culture that may contain exosomes, or may not contain exosomes but contain factors capable of stimulating de-differentiation. In such an embodiment, culture conditioned media may be concentrated by filtering/desalting means known in the art including use of Amicon filters with specific molecular weight cut-offs, and the cut-offs may select for molecular weights higher than 1 kDa to 50 kDa. Supernatant may alternatively be concentrated using means known in the art such as solid phase extraction using C18 cartridges (Mini-Spe-ed C18-14%, S.P.E. Limited, Concord ON). The cartridges are prepared by washing with methanol followed by deionized-distilled water. Up to 100 ml of de-differentiated fibroblast conditioned media supernatant may be passed through each of these specific cartridges before elution, and it is understood of one of skill in the art that larger cartridges may be used. After washing the cartridges material adsorbed is eluted with 3 ml methanol, evaporated under a stream of nitrogen, re-dissolved in a small volume of methanol, and stored at 4° C. Before testing the eluate for activity *in vitro,* the methanol is evaporated under nitrogen and replaced by culture medium. C18 cartridges may be used to adsorb small hydrophobic molecules from said dedifferentiated fibroblast conditioned supernatant, and allows for the elimination of salts and other polar contaminants. It may, however be desired to use other adsorption means in order to purify certain compounds from the supernatant. The concentrated supernatant may be assessed directly for biological activities useful for the practice of this disclosure, or may be further purified. Further purification may be performed using, for example, gel filtration using a Bio-Gel P-2 column with a nominal exclusion limit of 1800 Da (Bio-Rad, Richmond Calif.). Said column may be washed and pre-swelled in 20 mM Tris-HCl buffer, pH 7.2 (Sigma) and degassed by gentle swirling under vacuum. Bio-Gel P-2 material be packed into a 1.5x54 cm glass column and equilibrated with 3 column volumes of the same buffer. Dedifferentiated fibroblast cell supernatant concentrates extracted by C18 cartridge may be dissolved in 0.5 ml of 20 mM Tris buffer, pH 7.2 and run through the column. Fractions may be collected from the column and analyzed for biological activity. Other purification, fractionation, and identification means are known to one skilled in the art and include anionic exchange chromatography, gas chromatography, high performance liquid chromatography, nuclear magnetic resonance, and mass spectrometry. Administration of supernatant active fractions may be performed locally or systemically.

In particular embodiments, methods for treating disc degeneration include administration of conditioned media (or components therefrom) using processing methods as described herein.

### IV. Examples of Methods of Use

Embodiments of the disclosure include methods of delivering to an individual in need thereof an effective amount of conditioned media from culture of de-differentiated fibroblasts or one or more components therefrom.

In some embodiments, one or more agents known to inhibit apoptosis of nucleus pulposus cells are administered together with conditioned media, such agents include one or more of BMP-7 [56], bcl-2 gene therapy [57], platelet rich plasma [58], reseveratrol [59, 60], IGF-1 [61], SIRT-1 [62-67], transfection of MiR-27a [68], transfection of MiR-93 [69], transfection of MiR-494 [70], transfection of HIF-1 alpha [71], Pyrroloquinoline quinone [72], carboxymethylated chitosan [73], transfection of caveolin-1 [74], estradiol [75, 76], paeoniflorin [77], hepatocyte growth factor [78], vitamin C [79], transfection of survivin [80-82], transfection with RASS7 [83], PDGF-BB [84], metformin [85], hydrogen sulfide [86], angiopoietin [87], and/or gallic acid [88].

In some embodiments the disclosure, de-differentiated fibroblast conditioned media is utilized in conjunction with one or more therapeutic interventions known to inhibit apoptosis of nucleus pulposus cells, and such therapeutic interventions include hyperbaric oxygen [89, 90], adipose stem cell administration [91], bone marrow mesenchymal stem cell administration [92], fibroblast administration [93], and a combination thereof.

To administer the active ingredients of the composition of the disclosure by other than parenteral administration, for example, it may be necessary to coat the composition with, or co-administer the composition with, a material to prevent its inactivation. Enzyme inhibitors may be utilized and include pancreatic trypsin inhibitor, diisopropylfluorophosphate (DEP) and trasylol. Liposomes include water-in oil-in-water emulsions as well as conventional liposomes (Strejan et al., (1984) J. Neuroimmunol 7:27). The active compounds may also be administered parenterally or intraperitoneally. Dispersions can also be prepared in glycerol, liquid polyethylene glycols, and mixtures thereof and in oils. Under ordinary conditions of storage and use, these preparations may contain a preservative to prevent the growth of microorganisms.

The de-differentiated fibroblast media may be used for the basis of a pharmaceutical composition. The pharmaceutical composition(s) suitable for injectable use include sterile aqueous solutions (where water soluble) or dispersions and sterile powders for the extemporaneous preparation of sterile injectable solutions or dispersion. In all cases, the composition is sterile and is fluid to the extent that easy syringeability exists. It is stable under the conditions of manufacture and storage and must be preserved against the contaminating action of microorganisms such as bacteria and fungi. The pharmaceutically acceptable carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, asorbic acid, thimerosal, and the like. In many cases, it is useful to include isotonic agents, for example, sugars, polyalcohols such as mannitol, sorbitol, sodium chloride in the composition. Prolonged absorption of the injectable compositions can be brought about by including in the composition an agent that delays absorption, for example, aluminum monostearate and gelatin. Sterile injectable solutions for administration into the disc can be prepared by incorporating active compounds (for example, disc regenerative factors) in the required amount in an appropriate solvent with one or a combination of ingredients enumerated above, as required, followed by filtered sterilization. Generally, dispersions are prepared by incorporating the active compounds into a sterile vehicle which contains a basic dispersion medium and the required other ingredients from those enumerated above. In the case of sterile powders for the preparation of sterile injectable solutions, the methods of preparation are vacuum drying and freeze-drying that yields a powder of the active ingredient(s) plus any additional desired ingredient from a previously sterile-filtered solution thereof. When the active compounds (*e*.*g*., de-differentiated fibroblast conditioned media) are suitably protected, as described above, the composition may be orally administered, for example, with an inert diluent or an assimilable edible carrier.

Pharmaceutically acceptable carrier includes any and all solvents, dispersion media, coatings, antibacterial and antifungal agents, isotonic and absorption delaying agents, and the like. The use of such media and agents for pharmaceutically active substances is well known in the art. Except insofar as any conventional media or agent is incompatible with the active compound(s), use thereof in the therapeutic compositions is contemplated. Supplementary active compounds can also be incorporated into the compositions.

It is useful in particular embodiments to formulate compositions in dosage unit form for ease of administration and uniformity of dosage. Dosage unit form as used herein refers to physically discrete units suited as unitary dosages for the mammalian subjects to be treated; each unit containing a predetermined quantity of active compounds calculated to produce the desired therapeutic effect in association with the required pharmaceutical carrier. The specification for the dosage unit forms of the disclosure are dictated by and directly dependent on (a) the unique characteristics of the active compounds and the particular therapeutic effect to be achieved, and (b) the limitations inherent in the art of compounding such active compounds for the therapeutic treatment of individuals. Conventional procedures and ingredients for the selection and preparation of suitable formulations are described, for example, in Remington's Pharmaceutical Sciences (1990-18th edition) and in The United States Pharmacopeia The National Formulary (USP 24 NF19) published in 1999.

### V. General Embodiments

Methods and compositions related to preparation and/or use of fibroblasts cells as delivery agents may or may not have general elements as follows.

The amount of any types of cells for administration to an individual may depend on the type of disease to be treated, of the severity and stage of the disease, and/or of the type of cells to be injected for the treatment. The cells may be prepared for administration in a pharmaceutically acceptable carrier, for example a sterile saline isotonic solution. In some embodiments, the pharmaceutically acceptable carrier may comprise one or more additional agents, such as FAS ligand, IL-2R, IL-1 Ra, IL-2, IL-4, IL-8, IL-10, IL-20, IL-35, HLA-G, PD-L1, I-309, IDO, iNOS, CD200, Galectin 3, sCR1, arginase, PGE-2, aspirin, atorvastatin, fluvastatin, lovastatin, pravastatin, rosuvastatin, simvastatin, pitavastatin, n-acetylcysteine, rapamycin, IVIG, naltrexone, TGF-beta, VEGF, PDGF, CTLA-4, anti-CD45RB antibody, hydroxychloroquine, leflunomide, auranofin, dicyanogold, sulfasalazine, methotrexate, glucocorticoids, etanercept, adalimumab, abatacept, anakinra, certolizumab, Etanercept-szzs, golimumab, infliximab, rituximab, tocilizumab, cyclosporine, IFN-gamma, everolimus, rapamycin, VEGF, FGF-1, FGF-2, angiopoietin, HIF-1-alpha, or a combination thereof.

In one embodiment of the disclosure, fibroblasts are administered to a subject by any suitable route, including by injection, including in hypoxic areas. In other embodiments, given the ability of fibroblasts to home to cancer cells, the modified fibroblast cells may be provided systemically to an individual. Suitable routes include intramuscular, intravenous, subcutaneous, intrathecal, oral, intrarectal, intrathecal, intra-omental, intraventricular, intrahepatic, topical, and intrarenal.

In certain embodiments, fibroblasts may be derived from tissues comprising skin, heart, blood vessels, bone marrow, skeletal muscle, liver, pancreas, brain, adipose tissue, foreskin, placental, and/or umbilical cord, for example. In specific embodiments, the fibroblasts are placental, fetal, neonatal or adult or mixtures thereof.

The number of administrations of material to an individual will depend upon the factors described herein at least in part and may be optimized using routine methods in the art. In specific embodiments, a single administration is required. In other embodiments, a plurality of administration of cells is required. It should be appreciated that the system is subject to variables, such as the particular need of the individual, which may vary with time and circumstances, the rate of loss of the cellular activity as a result of loss of cells or activity of individual cells, and the like. Therefore, it is expected that each individual could be monitored for the proper dosage, and such practices of monitoring an individual are routine in the art.

In some embodiments, the cells are subjected to one or more media compositions that comprises, consists of, or consists essentially of Roswell Park Memorial Institute (RPMI-1640), Dublecco's Modified Essential Media (DMEM), Eagle's Modified Essential Media (EMEM), Optimem, Iscove's Media, or a combination thereof.

In one embodiment of the disclosure, the fibroblast cells are cultured *ex vivo* using means known in the art for preserving viability and proliferative ability of the cells. In specific embodiments for fibroblasts, there may be modification of known culture techniques to achieve one or more desired effects for the cells, such as to enhance homing capabilities, decrease visibility of fibroblasts to a recipient immune system, and so forth. In one embodiment, fibroblast cells are cultured in conditions that lack one or more xenogeneic components, such as fetal calf serum. In specific embodiments, the disclosure encompasses the substitution of fetal calf serum with human platelet rich plasma, platelet lysate, umbilical cord blood serum, autologous serum, and/or defined cytokine mixes as an additional feature, for example to reduce the immunogenicity of the fibroblast cells.

In some embodiments, fibroblasts (whether dedifferentiated or not) and/or compositions comprising the media and/or compositions comprising the exosomes are frozen and/or obtained from storage before use. In one example, protocols for freezing are used as described in the art, for example, freezing solutions include 5% DMSO, 30% FBS in alpha-MEM medium (Gibco-BRL, other solutions include human albumin 4.5% solution (ZENALB 4.5, Bio Products Laboratory) containing either no DMSO (D0) or increasing concentrations of DMSO (CryoSure-DMSO, WAK-Chemie Medical) from 0.5% to 20% (D0.5-D20, respectively). Prior to freezing, cells are harvested and washed with 1 × phosphate-buffered saline; then, cell pellets directly resuspended in 1 mL of freezing solution at concentrations of 1 × 10⁶ cells/mL, 5 × 10⁶ cells/mL or 10 × 10⁶ cells/mL, transferred into cryovials followed by placing the cryovials in an isopropanol freezing box (Nalgene cryo 1°C/min freezing container, Nalgene) for overnight freezing in a -80°C freezer (New Brunswick Scientific), and then stored in liquid nitrogen vapor (Taylor-Wharton) for at least 1 week and up to 3 weeks. Before use, the cells may be thawed by rapidly immersing the cryovials in a 37°C water bath with gentle shaking for 2 min, followed by transfering cells into 9 mL of warmed α-MEM for wash and cells pelleted by centrifugation at 1100 rpm for 5 min. The pelleted cells are then assessed for viability before administration. In some embodiments, migration ability and/or functionality of cells is tested.

In cases wherein recombination technology is employed, one or more types of the fibroblast cells (whether or not they are dedifferentiated) are manipulated to harbor one or more expression vectors that each encode one or more gene products of interest. A recombinant expression vector(s) can be introduced as one or more DNA molecules or constructs, where there may be at least one marker that will allow for selection of host cells that contain the vector(s). The vector(s) can be prepared in conventional ways, wherein the genes and regulatory regions may be isolated, as appropriate, ligated, cloned in an appropriate cloning host, and analyzed by sequencing or other convenient means. Particularly, using PCR, individual fragments including all or portions of a functional unit may be isolated, where in some cases one or more mutations may be introduced using "primer repair", ligation, *in vitro* mutagenesis, *etc.* as appropriate. The vector(s) once completed and demonstrated to have the appropriate sequences may then be introduced into the host cell by any convenient means. The constructs may be integrated and packaged into non-replicating, defective viral genomes like lentivirus, Adenovirus, Adeno-associated virus (AAV), Herpes simplex virus (HSV), or others, including retroviral vectors, for infection or transduction into cells. The vector(s) may include viral sequences for transfection, if desired. Alternatively, the construct may be introduced by fusion, electroporation, biolistics, transfection, lipofection, or the like. The host cells may be grown and expanded in culture before introduction of the vector(s), followed by the appropriate treatment for introduction of the vector(s) and integration of the vector(s). The cells are then expanded and screened by virtue of a marker present in the construct. Various markers that may be used successfully include hprt, neomycin resistance, thymidine kinase, hygromycin resistance, *etc.*

Any of the genes or gene products described herein, or active portions thereof, may be cloned into mammalian expression constructs comprising one or more promoter sequences enabling expression in cells such as the CMV promoter [Artuc et al., Exp. Dermatol. 1995, 4:317-21]. Examples of suitable constructs include, but are not limited to pcDNA3, pcDNA3.1 (+/-), pGL3, PzeoSV2 (+/-), pDisplay, pEF/myc/cyto, pCMV/myc/cyto each of which is commercially available, or the pSH expression vector which enables a regulated polynucleotide expression in human foreskin cells [Ventura and Villa, 1993, Biochem. Biophys. Commun. 192: 867-9]. Examples of retroviral vector and packaging systems are those sold by Clontech, San Diego, Calif., USA, including Retro-X vectors pLNCX and pLXSN, which permit cloning into multiple cloning sites and the transgene is transcribed from CMV promoter. Vectors derived from Mo-MuLV are also included such as pBabe, where the transgene will be transcribed from the 5'LTR promoter. After completing plasmid transfection fibroblasts are harvested by a means allowing for detachment from tissue culture plates, for example, by trypsinization and transferred to either a 6-well (Nunc, Denmark) or a 24-well plate (Nunc) for proliferation. Approximately 3 days post-transfection, the cell media is changed to media allow for proliferation and expansion of modified fibroblasts. One example is Neurobasal A (NBA) proliferation medium comprising Neurobasal-A (Invitrogen), 1% D-glucose (Sigma Aldrich), 1% Penicillin/Streptomycin/Glutamine (Invitrogen), 2% B27 supplement with Retinoic acid (Invitrogen), 0.2% EGF (Peprotech, USA), 0.08% FGF-2 (Peprotech), 0.2% Heparin (Sigma Aldrich, USA) and Valproic acid (Sigma-Aldrich) to a concentration of 1 µM. The media is then subsequently changed thrice weekly, and cells are re-plated regularly (for example, 2-8 times up to a maximum of weekly re-plating, becoming more regular as colonies began to develop) to remove non-reprogrammed cells until widespread colony formation is achieved.

In some instances, one or more agents may be introduced into the cells as an RNA molecule for transient expression. RNA can be delivered to any cells, including any modified cells, of the disclosure by various means including microinjection, electroporation, and lipid-mediated transfection, for example. In particular aspects, introduction of vector(s) into cells may occur *via* transposons. An example of a synthetic transposon for use is the Sleeping Beauty transposon that comprises an expression cassette including the angiogenic agent gene thereof. Alternatively, one may have a target site for homologous recombination, where it is desired that vector(s) be integrated at a particular locus using materials and methods as are known in the art for homologous recombination. For homologous recombination, one may use either OMEGA or O-vectors. See, for example, Thomas and Capecchi, 1987; Mansour, *et al.,* 1988; and Joyner, *et al.,* 1989.

The vector(s) may be introduced as a single DNA molecule encoding at least one agent (including one or more tumor inhibitory agents or functional fragments thereof) and optionally another polynucleotide (such as genes), or different DNA molecules having one or more polynucleotides (such as genes). The vector(s) may be introduced simultaneously or consecutively, each with the same or different markers. In an illustrative example, one vector would contain one or more agents (such as angiogenic agent(s)) under the control of particular regulatory sequences.

Vector(s) comprising useful elements such as bacterial or yeast origins of replication, selectable and/or amplifiable markers, promoter/enhancer elements for expression in prokaryotes or eukaryotes, *etc.* that may be used to prepare stocks of vector DNAs and for carrying out transfections are well known in the art, and many are commercially available.

In certain embodiments, it is contemplated that RNAs or proteinaceous sequences may be co-expressed with other selected RNAs or proteinaceous sequences in the same host cell. Co-expression may be achieved by co-transfecting the host cell with two or more distinct recombinant vectors. Alternatively, a single recombinant vector may be constructed to include multiple distinct coding regions for RNAs, which could then be expressed in host cells transfected with the single vector.

In some situations, it may be desirable to kill the modified cells, such as when the object is to terminate the treatment, the cells become neoplastic, in research where the absence of the cells after their presence is of interest, and/or another event. For this purpose one can provide for the expression of certain gene products in which one can kill the modified cells under controlled conditions, such as a suicide gene. Suicide genes are known in the art, *e.g.* the iCaspase9 system in which a modified form of caspase 9 is dimerizable with a small molecule, *e.g.* AP1903. See, *e.g.,* Straathof et al., Blood 105:4247-4254 (2005).

### VI. Kits of the Disclosure

Any of the cellular and/or non-cellular compositions described herein or similar thereto may be comprised in a kit. In a non-limiting example, one or more reagents for use in methods for preparing fibroblasts may be comprised in a kit. Such reagents may include cells, vectors, one or more growth factors, vector(s) one or more costimulatory factors, media, enzymes, buffers, nucleotides, salts, primers, and so forth. The kit components are provided in suitable container means.

Some components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits of the present disclosure also will typically include a means for containing the components in close confinement for commercial sale. Such containers may include injection or blow molded plastic containers into which the desired vials are retained.

When the components of the kit are provided in one and/or more liquid solutions, the liquid solution is an aqueous solution, with a sterile aqueous solution being particularly useful. In some cases, the container means may itself be a syringe, pipette, and/or other such like apparatus, or may be a substrate with multiple compartments for a desired reaction.

Some components of the kit may be provided as dried powder(s). When reagents and/or components are provided as a dry powder, the powder can be reconstituted by the addition of a suitable solvent. It is envisioned that the solvent may also be provided in another container means. The kits may also comprise a second container means for containing a sterile acceptable buffer and/or other diluent.

In specific embodiments, reagents and materials include primers for amplifying desired sequences, nucleotides, suitable buffers or buffer reagents, salt, and so forth, and in some cases the reagents include apparatus or reagents for isolation of a particular desired cell(s).

In particular embodiments, there are one or more apparatuses in the kit suitable for extracting one or more samples from an individual. The apparatus may be a syringe, fine needles, scalpel, and so forth.

### EXAMPLES

The following examples are included to demonstrate particular embodiments of the disclosure. It should be appreciated by those of skill in the art that the techniques disclosed in the examples that follow represent techniques discovered by the inventor to function well in the practice of the methods of the disclosure, and thus can be considered to constitute particular modes for its practice. However, those of skill in the art should, in light of the present disclosure, appreciate that many changes can be made in the specific embodiments which are disclosed and still obtain a like or similar result without departing from the spirit and scope of the disclosure.

### EXAMPLE 1

### AN EXAMPLE OF GENERATION OF DEDIFFERENTIATED FIBROBLASTS

### Donor sample acquisition and cell extraction

Donors skin biopsy tissue is extracted under aseptic conditions. Fibroblasts are liberated from the skin biopsy by an enzymatic digestion process. All procedures requiring open manipulation are performed in Class 100 Laminar Flow Biosafety Cabinets (LFBSCs); all subsequent procedures are performed in a well maintained Class 10,000 suite. The biopsy transport container (hypothermosol bottle) is removed from the shipping container and wiped with 70% ETOH before being brought into the LFBSC. The biopsy is transferred into a Petri dish. Extraneous non-skin tissue is dissected from the biopsy and the biopsy weight is obtained. The skin biopsy is aseptically minced using scalpels and/or scissors until all of the tissue is approximately 1 mm³ in size. The minced tissue is then transferred into 50 ml conical tubes containing pre-formulated enzymatic digestion solution consisting of a mixture of collagenase in HBSS (Vitacyte Inc, catalogue #005-1190). The conical tubes are capped and placed at approximately 37°C in a dry bath. The digestion is allowed to proceed for up to 120 minutes, with manual swirling of the tubes approximately every 10-15 minutes. While the digestion is proceeding the LFBSC is cleared of the biopsy preparation materials and prepared for subsequent processing.

### Expansion of fibroblasts

On completion of the digestion period, the tissue and enzyme containing conical tubes are removed from the bath and wiped with 70% ETOH prior to entering the LFBSC. The digested tissue is gravity filtered through sterile nylon filters placed on top of conical tubes to remove undigested tissue. The digested tissue is concentrated *via* centrifugation and the cellular debris and the digestive enzymes are removed in the supernatant. The resulting isolated fibroblasts are then seeded into a Cell Factory by sterile welding in culture media supplemented with irradiated FBS. Sterile welding is performed per standard operating procedures utilizing validated equipment. The cell factory is labeled with the patient specific lot number and placed into an incubator at approximately 37°C. Fibroblasts lots utilize physical segregation and line clearance procedures to prevent cross contamination between lots. Cultures are fed with the media every 3-4 days. The old media is drained by gravity through tubing sets into a waste collection bag. A new bag of media is welded onto the factory and allowed to drain into the factory by gravity. The confluence of the culture is monitored routinely. The culture is passaged, harvested, washed, and replated when the confluence is estimated to be between 60% and 100% of the culture surface.

### Cell Passaging

Passaging is accomplished by first washing the factories with HBSS to remove culture media. Then a non-animal derived trypsin-like solution (TrypLE Select - Invitrogen) is applied to the culture and the cells are observed by microscope for detachment from the culture surface. The cell factory is trypsinized for approximately 15 minutes. The cell factory is then rinsed with media utilizing tubing sets and bags to remove all detached cells and the cells are washed *via* centrifugation in a bag. The system remains closed during centrifugation and resuspension by utilizing sterile welding of bags and tubing sets. Following re-suspension in culture media a sample is removed via sterile welding with a sampling syringe and the cells are counted. The results of the cell count and viability determination are documented in the batch record.

The cells are then replated into 10-layer Cell Factories (10LCF). Typical cell yields lead to seeding the 10LCF at densities between 1000 and 9000 cells per cm². The cultures are processed as described above. Again, individual fibroblast lots are subjected to physical segregation and line clearance procedures are performed between individual lot processing activities.

In particular embodiments, substantial numbers of fibroblasts are required for therapeutic implantation. The initial isolation produces a relatively small amount of fibroblasts that must be expanded. The expansion to potential therapeutic doses requires 30 - 60 days of culturing and multiple passages (up to 7) of the cell culture. The fibroblast culture system is comprised of 1LCF (632 cm² of culture surface area) at the initial stages and transitions to 10LCF (6320 cm² of culture surface area) as the expansion continues. Antibiotics are removed early in the process (day 5-6 of a 30 - 60 day process), allowing several media changes and several passages in antibiotic free media.

Cells are cultured in 5% (hypoxia) O₂ in the presence of 0.5 mM VPA. A cell culture medium is Media 106 growth medium, which is a sterile liquid culture medium containing essential and non-essential amino acids, vitamins, other organic compounds, trace minerals, and inorganic salts, for the growth of normal human fibroblast cells. The medium is bicarbonate buffered and has a pH of 7.4+/- 0.2. It is used in a 5% CO₂ humidified environment. The media is provided by Thermo Fisher as a membrane filtered aseptically processed product packaged in sterile containers. The minimum sterility assurance level ("SAL") is 10⁻³, based on the number of media vials filled and the observed lack of microbial growth as described in FDA's "Guideline on Sterile Drug Products Produced by Aseptic Processing."

### Determination of Dedifferentiation Status

In some embodiments, passaged cells are defined as "dedifferentiated" if expression of OCT-4 is detected. The quantification of OCT-4 is performed using fix-and-perm intracellular staining purchased from R&D Systems. Assessment of expression is performed using flow cytometry. There is consistent induction of OCT-4 expression by culture in the combination of hypoxia and valproic acid, such as when fibroblasts are cultured under these conditions for more than 4 days.

### EXAMPLE 2

### CONCENTRATION OF THERAPEUTIC FRACTION FROM CONDITIONED MEDIA

Culture media is obtained prior to passaging of cells and cellular debris is removed by centrifugation at 400 g for 20 minutes. Supernatant extracted after this centrifugation step is subsequently spun at 70,000 g for 8 hours in order to pellet exosomes and other microvesicles. Purified particles are subsequently diluted in cell culture media and protein quantification is performed in a representative aliquot using the Bradfort Assay.

### EXAMPLE 3

### MODIFICATION OF NUCLEUS PULPOSUS CELLS BY CONDITIONED MEDIA

Nucleus pulposus (NP) tissue samples were obtained from patients undergoing spine surgery because of burst thoracolumbar fracture. The experimental protocol was approved by the institutional review board with informed consent from the patients. The NP tissues were treated with 0.25% pronase (Sigma-Aldrich, Louis, MO, USA) for 30 min. and 0.2% collagenase type II (Invitrogen, Carlsbad, CA, USA) for 4 hrs at 37°C. The digest was filtered through a 70-µm pore size mesh and then cultured in Dulbecco's modified Eagle's medium (DMEM; Gibco, Grand Island, NY, USA) with 10% fetal bovine serum (FBS; Invitrogen), 1% penicillin-streptomycin (Sigma-Aldrich), 2 mM glutamine (Sigma-Aldrich) and 50 µg/mL L-ascorbic acid (Sigma-Aldrich) in T25 flasks at 37°C in 5% CO₂. When grown to confluence, the cells were digested by 0.25% trypsin/1 mM EDTA and passed into bigger flasks for expansion. The nucleus pulposus cells (NPCs) from passage 4 or 5 were plated into experimental plates for all of the experiments.

NPCs were plated into 6-well plates at a density of 1 × 10⁵ cells per well. To test the apoptosis-inducing effect of TNF-α on cells, NPCs were treated with 0, 5, 10 or 30 ng/ml TNF-α (Sigma-Aldrich) for 12 hrs. To assess the anti-apoptotic effect of dedifferentiated fibroblast exosomes, conventional fibroblast exosomes, and control fetal calf serum exosomes.

Following treatment, NPC apoptosis rates were evaluated by flow cytometry using an Annexin V/PI apoptosis detection kit (BD Biosciences, Franklin Lakes, NJ, USA). NPCs were washed twice with PBS, resuspended in binding buffer and incubated with 5 µl FITC-Annexin V and 5 µl PI for 15 min. at room temperature. Staining cells were analyzed using the FACScan flow cytometry system (Becton Dickinson, San Diego, CA, USA).

As seen in FIG. 1, exosomes from fibroblasts inhibited TNF-alpha induced apoptosis, whereas exosomes from dedifferentiated fibroblasts even more potently inhibited suppressed apoptosis. For each of the four groups of bars, the far left bar is "blue", the second to the left is "orange", the second from the right is "gray", and the right is "dark yellow." Blue bars are saline control, orange bars are from fibroblasts, grey bars are from dedifferentiated fibroblasts (hypoxia and valproic acid) and dark yellow bars are fetal calf serum exosomes.

Nucleus pulposus cells where cultured as described and treated with IGF-1 as a mitogen. Proliferation after 48 hours of culture was assessed by thymidine incorporation by pulsing with 1 microCurie per ml of tritiated thymidine. As seen in FIG. 2, exosomes from fibroblasts inhibited stimulated NP cell proliferation, whereas exosomes from dedifferentiated fibroblasts even more potently stimulatory. For each of the four groups of bars, the far left bar is "blue", the second to the left is "orange", the second from the right is "gray", and the right is "dark yellow." Blue bars are saline control, orange bars are from fibroblasts, grey bars are from dedifferentiated fibroblasts (hypoxia and valproic acid) and dark yellow bars are fetal calf serum exosomes. Stimulation of proliferation was enhanced by addition of IGF-1 to the culture.

### REFERENCES

All publications mentioned in the specification are indicative of the level of those skilled in the art to which the invention pertains. All publications are herein incorporated by reference to the same extent as if each individual publication was specifically and individually indicated to be incorporated by reference.
1. Singh, K., et al., Age-related changes in the extracellular matrix of nucleus pulposus and anulus fibrosus of human intervertebral disc. Spine (Phila Pa 1976), 2009. 34(1): p. 10-6.
2. Haefeli, M., et al., The course of macroscopic degeneration in the human lumbar intervertebral disc. Spine (Phila Pa 1976), 2006. 31(14): p. 1522-31.
3. Cs-Szabo, G., et al., Changes in mRNA and protein levels of proteoglycans of the anulus fibrosus and nucleus pulposus during intervertebral disc degeneration. Spine (Phila Pa 1976), 2002. 27(20): p. 2212-9.
4. Boos, N., et al., Classification of age-related changes in lumbar intervertebral discs: 2002 Volvo Award in basic science. Spine (Phila Pa 1976), 2002. 27(23): p. 2631-44.
5. Le Maitre, C.L., A.J. Freemont, and J.A. Hoyland, Localization of degradative enzymes and their inhibitors in the degenerate human intervertebral disc. J Pathol, 2004. 204(1): p. 47-54.
6. Patel, K.P., et al., Aggrecanases and aggrecanase-generated fragments in the human intervertebral disc at early and advanced stages of disc degeneration. Spine (Phila Pa 1976), 2007. 32(23): p. 2596-603.
7. Fukuta, S., et al., Abundance of calpain and aggrecan-cleavage products of calpain in degenerated human intervertebral discs. Osteoarthritis Cartilage, 2011. 19(10): p. 1254-62.
8. Lotz, J.C., et al., Compression-induced degeneration of the intervertebral disc: an in vivo mouse model and finite-element study. Spine (Phila Pa 1976), 1998. b(23): p. 2493-506.
9. Heyde, C.E., et al., Trauma induces apoptosis in human thoracolumbar intervertebral discs. BMC Clin Pathol, 2006. 6: p. 5.
10. Kim, K.W., et al., An autocrine or paracrine Fas-mediated counterattack: a potential mechanism for apoptosis of notochordal cells in intact rat nucleus pulposus. Spine (Phila Pa 1976), 2005. 30(11): p. 1247-51.
11. Zhao, C.Q., et al., The cell biology of intervertebral disc aging and degeneration. Ageing Res Rev, 2007. 6(3): p. 247-61.
12. Wang, D.L., S.D. Jiang, and L.Y. Dai, Biologic response of the intervertebral disc to static and dynamic compression in vitro. Spine (Phila Pa 1976), 2007. 32(23): p. 2521-8.
13. Wang, J., et al., The expression of Fas ligand on normal and stabbed-disc cells in a rabbit model of intervertebral disc degeneration: a possible pathogenesis. J Neurosurg Spine, 2007. 6(5): p. 425-30.
14. Wang, H.Q., et al., Deregulated miR-155 promotes Fas-mediated apoptosis in human intervertebral disc degeneration by targeting FADD and caspase-3. J Pathol, 2011. 225(2): p. 232-42.
15. Liu, Z.H., et al., FasL expression on human nucleus pulposus cells contributes to the immune privilege of intervertebral disc by interacting with immunocytes. Int J Med Sci, 2013. 10(8): p. 1053-60.
16. Zhang, L., et al., The occurrence and regional distribution of DR4 on herniated disc cells: a potential apoptosis pathway in lumbar intervertebral disc. Spine (Phila Pa 1976), 2008. 33(4): p. 422-7.
17. Wang, H., et al., Role of death receptor, mitochondrial and endoplasmic reticulum pathways in different stages of degenerative human lumbar disc. Apoptosis, 2011. 16(10): p. 990-1003.
18. Johnson, Z.I., et al., Disc in flames: Roles of TNF-alpha and IL-1beta in intervertebral disc degeneration. Eur Cell Mater, 2015. 30: p. 104-16; discussion 116-7.
19. Xu, F., et al., Bioinformatics analysis of molecular mechanisms involved in intervertebral disc degeneration induced by TNF-alpha and IL-1beta. Mol Med Rep, 2016. 13(3): p. 2925-31.
20. Wang, B., et al., MiR-138-5p promotes TNF-alpha-induced apoptosis in human intervertebral disc degeneration by targeting SIRT1 through PTEN/PI3K/Akt signaling. Exp Cell Res, 2016. 345(2): p. 199-205.
21. Yu, H. and Y. Zhu, Expression of ADAMTS-7 and ADAMTS-12 in the nucleus pulposus during degeneration of rat caudal intervetebral disc. J Vet Med Sci, 2012. 74(1): p. 9-15.
22. Wang, S.S., et al., IL-17A enhances ADAMTS-7 expression through regulation of TNF-alpha in human nucleus pulposus cells. J Mol Histol, 2015. 46(6): p. 475-83.
23. Wang, Z., et al., Interleukin-2 is upregulated in patients with a prolapsed lumbar intervertebral disc and modulates cell proliferation, apoptosis and extracellular matrix metabolism of human nucleus pulposus cells. Exp Ther Med, 2015. 10(6): p. 2437-2443.
24. Vrana, K.E., et al., Nonhuman primate parthenogenetic stem cells. Proc Natl Acad Sci U S A, 2003. 100 Suppl 1: p. 11911-6.
25. Sanchez-Pemaute, R., et al., Long-term survival of dopamine neurons derived from parthenogenetic primate embryonic stem cells (cyno-1) after transplantation. Stem Cells, 2005. 23(7): p. 914-22.
26. Cibelli, J.B., K. Cunniff, and K.E. Vrana, Embryonic stem cells from parthenotes. Methods Enzymol, 2006. 418: p. 117-35.
27. Revazova, E.S., et al., Patient-specific stem cell lines derived from human parthenogenetic blastocysts. Cloning Stem Cells, 2007. 9(3): p. 432-49.
28. de Fried, E.P., et al., Human parthenogenetic blastocysts derived from noninseminated cryopreserved human oocytes. Fertil Steril, 2008. 89(4): p. 943-7.
29. French, A.J., S.H. Wood, and A.O. Trounson, Human therapeutic cloning (NTSC): applying research from mammalian reproductive cloning. Stem Cell Rev, 2006. 2(4): p. 265-76.
30. Lin, G., et al., A highly homozygous and parthenogenetic human embryonic stem cell line derived from a one-pronuclear oocyte following in vitro fertilization procedure. Cell Res, 2007. 17(12): p. 999-1007.
31. Revazova, E.S., et al., HLA homozygous stem cell lines derived from human parthenogenetic blastocysts. Cloning Stem Cells, 2008. 10(1): p. 11-24.
32. De Sousa, P.A. and I. Wilmut, Human parthenogenetic embryo stem cells: appreciating what you have when you have it. Cell Stem Cell, 2007. 1(3): p. 243-4.
33. Wun, I.C. and R.E. Dittman, Human somatic cell nuclear transfer. Chin J Physiol, 2008. 51(4): p. 208-13.
34. Taupin, P., Parthenogenetically activated human oocytes and parthenogenetic embryonic stem cells: US20100233143. Expert Opin Ther Pat, 2011. 21(8): p. 1281-3.
35. Wei, Q., et al., Derivation of rhesus monkey parthenogenetic embryonic stem cells and its microRNA signature. PLoS One, 2011. 6(9): p. e25052.
36. Yabuuchi, A., H. Rehman, and K. Kim, Histocompatible parthenogenetic embryonic stem cells as a potential source for regenerative medicine. J Mamm Ova Res, 2012. 29(1): p. 17-21.
37. Daughtry, B. and S. Mitalipov, Concise review: parthenote stem cells for regenerative medicine: genetic, epigenetic, and developmental features. Stem Cells Transl Med, 2014. 3(3): p. 290-8.
38. Espejel, S., et al., Brief report: Parthenogenetic embryonic stem cells are an effective cell source for therapeutic liver repopulation. Stem Cells, 2014. 32(7): p. 1983-8.
39. Cervera, R.P. and M. Stojkovic, Human embryonic stem cell derivation and nuclear transfer: impact on regenerative therapeutics and drug discovery. Clin Pharmacol Ther, 2007. 82(3): p. 310-5.
40. De Sousa, P.A., et al., Clinically failed eggs as a source of normal human embryo stem cells. Stem Cell Res, 2009. 2(3): p. 188-97.
41. Takahashi, K. and S. Yamanaka, Induction of pluripotent stem cells from mouse embryonic and adult fibroblast cultures by defined factors. Cell, 2006. 126(4): p. 663-76.
42. Park, I.H., et al., Reprogramming of human somatic cells to pluripotency with defined factors. Nature, 2008. 451(7175): p. 141-6.
43. Chhabra, A., Derivation of Human Induced Pluripotent Stem Cell (iPSC) Lines and Mechanism of Pluripotency: Historical Perspective and Recent Advances. Stem Cell Rev, 2017.
44. Shi, Y., et al., Induced pluripotent stem cell technology: a decade of progress. Nat Rev Drug Discov, 2017. 16(2): p. 115-130.
45. Kele, M., et al., Generation of human iPS cell line CTL07-IIfrom human fibroblasts, under defined and xeno-free conditions. Stem Cell Res, 2016. 17(3): p. 474-478.
46. Obokata, H., et al., Bidirectional developmental potential in reprogrammed cells with acquired pluripotency. Nature, 2014. 505(7485): p. 676-80.
47. Zhou, Q., et al., A comparative approach to somatic cell nuclear transfer in the rhesus monkey. Hum Reprod, 2006. 21(10): p. 2564-71.
48. Hall, V.J., et al., Developmental competence of human in vitro aged oocytes as host cells for nuclear transfer. Hum Reprod, 2007. 22(1): p. 52-62.
49. Sung, L.Y., et al., Efficient derivation of embryonic stem cells from nuclear transfer and parthenogenetic embryos derived from cryopreserved oocytes. Cell Reprogram, 2010. 12(2): p. 203-11.
50. Collas, P. and C.K. Taranger, Epigenetic reprogramming of nuclei using cell extracts. Stem Cell Rev, 2006. 2(4): p. 309-17.
51. Collas, P. and C.K. Taranger, Toward reprogramming cells to pluripotency. Ernst Schering Res Found Workshop, 2006(60): p. 47-67.
52. Collas, P., et al., On the way to reprogramming cells to pluripotency using cell-free extracts. Reprod Biomed Online, 2006. 12(6): p. 762-70.
53. Liu, Y., M.N. Rahaman, and B.S. Bal, Modulating notochordal differentiation of human induced pluripotent stem cells using natural nucleus pulposus tissue matrix. PLoS One, 2014. 9(7): p. e100885.
54. Liu, Y., et al., Native nucleus pulposus tissue matrix promotes notochordal differentiation of human induced pluripotent stem cells with potential for treating intervertebral disc degeneration. J Biomed Mater Res A, 2015. 103(3): p. 1053-9.
55. Chen, J., et al., Differentiation of mouse induced pluripotent stem cells (iPSCs) into nucleus pulposus-like cells in vitro. PLoS One, 2013. 8(9): p. e75548.
56. Wei, A., et al., Bone morphogenetic protein-7 protects human intervertebral disc cells in vitro from apoptosis. Spine J, 2008. 8(3): p. 466-74.
57. Sudo, H. and A. Minami, Regulation of apoptosis in nucleus pulposus cells by optimized exogenous Bcl-2 overexpression. J Orthop Res, 2010. 28(12): p. 1608-13.
58. Sawamura, K., et al., Characterization of in vivo effects of platelet-rich plasma and biodegradable gelatin hydrogel microspheres on degenerated intervertebral discs. Tissue Eng Part A, 2009. 15(12): p. 3719-27.
59. Li, X., et al., The action of resveratrol, a phytoestrogen found in grapes, on the intervertebral disc. Spine (Phila Pa 1976), 2008. 33(24): p. 2586-95.
60. Yang, S.D., et al., Combined effect of 17beta-estradiol and resveratrol against apoptosis induced by interleukin-1beta in rat nucleus pulposus cells via PI3K/Akt/caspase-3 pathway. PeerJ, 2016. 4: p. e1640.
61. Zhang, C.C., et al., Effects of IGF-1 on IL-1beta-induced apoptosis in rabbit nucleus pulposus cells in vitro. Mol Med Rep, 2013. 7(2): p. 441-4.
62. Wang, D., et al., SIRT1 inhibits apoptosis of degenerative human disc nucleus pulposus cells through activation of Akt pathway. Age (Dordr), 2013. 35(5): p. 1741-53.
63. Jiang, W., et al., SIRT1 protects against apoptosis by promoting autophagy in degenerative human disc nucleus pulposus cells. Sci Rep, 2014. 4: p. 7456.
64. Miyazaki, S., et al., Recombinant human SIRT1 protects against nutrient deprivation-induced mitochondrial apoptosis through autophagy induction in human intervertebral disc nucleus pulposus cells. Arthritis Res Ther, 2015. 17: p. 253.
65. Shen, J., et al., SIRT1 Inhibits the Catabolic Effect of IL-1beta Through TLR2/SIRT1/NF-kappaB Pathway in Human Degenerative Nucleus Pulposus Cells. Pain Physician, 2016. 19(1): p. E215-26.
66. Wang, X., et al., SIRT1 expression is refractory to hypoxia and inflammatory cytokines in nucleus pulposus cells: Novel regulation by HIF-1alpha and NF-kappaB signaling. Cell Biol Int, 2016. 40(6): p. 716-26.
67. Guo, J., et al., Role of Sirt1 Plays in Nucleus Pulposus Cells and Intervertebral Disc Degeneration. Spine (Phila Pa 1976), 2017. 42(13): p. E757-E766.
68. Liu, G., et al., MiR-27a regulates apoptosis in nucleus pulposus cells by targeting PI3K. PLoS One, 2013. 8(9): p. e75251.
69. Jing, W. and W. Jiang, MicroRNA-93 regulates collagen loss by targeting MMP3 in human nucleus pulposus cells. Cell Prolif, 2015. 48(3): p. 284-92.
70. Wang, T., et al., MicroRNA-494 inhibition protects nucleus pulposus cells from TNF-alpha-induced apoptosis by targeting JunD. Biochimie, 2015. 115: p. 1-7.
71. Merceron, C., et al., Loss of HIF-1alpha in the notochord results in cell death and complete disappearance of the nucleus pulposus. PLoS One, 2014. 9(10): p. e110768.
72. Yang, L., et al., Pyrroloquinoline quinone protects nucleus pulposus cells from hydrogen peroxide-induced apoptosis by inhibiting the mitochondria-mediated pathway. Eur Spine J, 2015. 24(8): p. 1702-10.
73. He, B., et al., Protective effect of carboxymethylated chitosan on hydrogen peroxide-induced apoptosis in nucleus pulposus cells. Mol Med Rep, 2015. 11(3): p. 1629-38.
74. Bach, F.C., et al., Increased caveolin-1 in intervertebral disc degeneration facilitates repair. Arthritis Res Ther, 2016. 18: p. 59.
75. Yang, S.D., et al., 17beta-estradiolprotects against apoptosis induced by interleukin-1beta in rat nucleus pulposus cells by down-regulating MMP-3 and MMP-13. Apoptosis, 2015. 20(3): p. 348-57.
76. Wei, A., et al., Expression and functional roles of estrogen receptor GPR30 in human intervertebral disc. J Steroid Biochem Mol Biol, 2016. 158: p. 46-55.
77. Shi, L., et al., Paeoniflorin inhibits nucleus pulposus cell apoptosis by regulating the expression of Bcl-2 family proteins and caspase-9 in a rabbit model of intervertebral disc degeneration. Exp Ther Med, 2015. 10(1): p. 257-262.
78. Ishibashi, H., et al., Hepatocyte growth factor/c-met promotes proliferation, suppresses apoptosis, and improves matrix metabolism in rabbit nucleus pulposus cells in vitro. J Orthop Res, 2016. 34(4): p. 709-16.
79. Dai, L., et al., [EFFECT OF VITAMIN C ON APOPTOSIS OF NUCLEUS PULPOSUS CELLS INDUCED BY TUMOR NECROSIS FACTOR a AND SERUM DEPRIVATION]. Zhongguo Xiu Fu Chong Jian Wai Ke Za Zhi, 2015. 29(4): p. 490-7.
80. Lin, Y., et al., Survivin is expressed in degenerated nucleus pulposus cells and is involved in proliferation and the prevention of apoptosis in vitro. Mol Med Rep, 2016. 13(1): p. 1026-32.
81. Yue, B., et al., Survivin-TGFB3-TIMP1 Gene Therapy Via Lentivirus Vector Slows the Course of Intervertebral Disc Degeneration in an In Vivo Rabbit Model. Spine (Phila Pa 1976), 2016. 41(11): p. 926-34.
82. Yue, B., et al., Effect of Survivin gene therapy via lentivirus vector on the course of intervertebral disc degeneration in an in vivo rabbit model. Mol Med Rep, 2016. 14(5): p. 4593-4598.
83. Liu, Z.H., et al., RASSF7 expression and its regulatory roles on apoptosis in human intervertebral disc degeneration. Int J Clin Exp Pathol, 2015. 8(12): p. 16097-103.
84. Paglia, D.N., et al., PDGF-BB Delays Degeneration of the Intervertebral Discs in a Rabbit Preclinical Model. Spine (Phila Pa 1976), 2016. 41(8): p. E449-58.
85. Chen, D., et al., Metformin protects against apoptosis and senescence in nucleus pulposus cells and ameliorates disc degeneration in vivo. Cell Death Dis, 2016. 7(10): p. e2441.
86. Xu, D., et al., Hydrogen sulfide protects against endoplasmic reticulum stress and mitochondrial injury in nucleus pulposus cells and ameliorates intervertebral disc degeneration. Pharmacol Res, 2017. 117: p. 357-369.
87. Wang, K., et al., The role of angiopoietin-2 in nucleus pulposus cells during human intervertebral disc degeneration. Lab Invest, 2017. 97(8): p. 971-982.
88. Huang, Y., et al., Gallic acid inhibits the release of ADAMTS4 in nucleus pulposus cells by inhibiting p65 phosphorylation and acetylation of the NF-kappaB signaling pathway. Oncotarget, 2017. 8(29): p. 47665-47674.
89. Wang, I.C., et al., Effect of hyperbaric oxygenation on intervertebral disc degeneration: an in vitro study with human lumbar nucleus pulposus. Spine (Phila Pa 1976), 2011. 36(23): p. 1925-31.
90. Niu, C.C., et al., Hyperbaric oxygen treatment suppresses MAPK signaling and mitochondrial apoptotic pathway in degenerated human intervertebral disc cells. J Orthop Res, 2013. 31(2): p. 204-9.
91. Sun, Z., et al., Adipose-derived stromal cells protect intervertebral disc cells in compression: implications for stem cell regenerative disc therapy. Int J Biol Sci, 2015. 11(2): p. 133-43.
92. Hu, J., et al., An in vitro investigation into the role of bone marrowderived mesenchymal stem cells in the control of disc degeneration. Mol Med Rep, 2015. 12(4): p. 5701-8.
93. Ural, I.H., et al., Fibroblast Transplantation Results to the Degenerated Rabbit Lumbar Intervertebral Discs. Open Orthop J, 2017. 11: p. 404-416.

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

### ASPECTS OF THE INVENTION

1. A method of treating or preventing disc degeneration in an individual, comprising the step of providing to the individual an effective amount of conditioned media from culture of de-differentiated fibroblasts or one or more components therefrom.
2. The method of aspect 1, wherein the conditioned media comprises exosomes.
3. The method of aspect 2, wherein the exosomes express markers selected from the group consisting of a) CD63; b) CD9; c) MHC I; d) CD56; and e) a combination thereof.
4. The method of any one of aspects 1-3, further comprising the step of de-differentiating the fibroblasts to produce de-differentiated fibroblasts.
5. The method of aspect 4, wherein the step of de-differentiating the fibroblasts to produce de-differentiated fibroblasts occurs in culture.
6. The method of aspect 4 or 5, wherein the de-differentiated fibroblasts are produced upon exposure to stem cells and/or cytoplasm from stem cells.
7. The method of aspect 6, wherein the stem cells are pluripotent stem cells selected from the group consisting of a) parthenogenic stem cells; b) embryonic stem cells; c) inducible pluripotent stem cells; d) somatic cell nuclear transfer derived stem cells; e) Stimulus-triggered acquisition of pluripotency (STAP); and f) a combination thereof.
8. The method of any one of aspects 4-7, wherein the de-differentiated fibroblasts are produced upon exposure to hypoxia.
9. The method of any one of aspects 4-8, wherein the de-differentiated fibroblasts are produced upon exposure to one or more histone deacetylase inhibitors.
10. The method of aspect 9, wherein the histone deacetylase inhibitor is selected from the group consisting of a) valproic acid; b) trichostatin A; c) phenylbutyrate; d) vorinostat; e) belinostat; f) LAQ824; g) panobinostat; h) entinostat; i) CI994; j) mocetinostat; k) sulforaphane; and l) a combination thereof.
11. The method of any one of aspects 4-10, wherein the de-differentiated fibroblasts are produced upon exposure to one or more DNA methyltransferase inhibitors.
12. The method of aspect 11, wherein the DNA methyltransferase inhibitor is selected from the group consisting of a) decitabine; b) 5-azacytidine; c) Zebularine; d) RG-108; e) procaine hydrochloride; f) Procainamide hydrochloride; g) Hydralazine hydrochloride; h) Epigallocatechin gallate; i) Chlorogenic acid; j) Caffeic acid; and k) a combination thereof.
13. The method of any one of aspects 4-12, wherein the de-differentiated fibroblasts are produced upon exposure to 2%-8%, 2%-7%, 2%-6%, 2%-5%, 2%-4%, 2%-3%, 3%-8%, 3%-7%, 3%-6%, 3%-5%, 3%-4%, 4%-8%, 4%-7%, 4%-6%, 4%-5%, 5%-8%, 5%-7%, 5%-6%, 6%-8%, 6%-7%, or 7%-8% oxygen.
14. The method of any one of aspects 1-13, wherein exosomes are obtained from de-differentiated fibroblasts.
15. The method of aspect 14, wherein the exosomes are purified from culture of the de-differentiated fibroblasts using anion exchange chromatography, high performance liquid chromatography (HPLC), or both.
16. The method of any one of aspects 1-15, comprising administering to the individual one or more of hyperbaric oxygen, adipose stem cell administration, bone marrow mesenchymal stem cell administration, fibroblast administration, and a combination thereof.
17. A composition comprising conditioned media from culture of de-differentiated fibroblasts or one or more components therefrom.
18. The composition of aspect 17, wherein the conditioned media comprises exosomes.
19. The composition of aspect 18, wherein the exosomes express markers selected from the group consisting of a) CD63; b) CD9; c) MHC I; d) CD56; and e) a combination thereof.

## Claims

1. Conditioned media from culture of de-differentiated fibroblasts for use in a method of treating or preventing disc degeneration in an individual, comprising the step of providing to the individual an effective amount of the conditioned media, wherein said de-differentiated fibroblasts are produced upon exposure to one or more histone deacetylase inhibitors.

2. The conditioned media for use according to claim 1, wherein the conditioned media comprises exosomes.

3. The conditioned media for use according to claim 2, wherein the exosomes express markers selected from the group consisting of a) CD63; b) CD9; c) MHC I; d) CD56; and e) a combination thereof.

4. The conditioned media for use according to any one of claims 1-3, the method further comprising the step of de-differentiating the fibroblasts to produce de-differentiated fibroblasts, optionally wherein the step of de-differentiating the fibroblasts to produce de-differentiated fibroblasts occurs in culture.

5. The conditioned media for use according to claim 4, wherein the de-differentiated fibroblasts are produced upon exposure to stem cells and/or cytoplasm from stem cells, optionally wherein the stem cells are pluripotent stem cells selected from the group consisting of a) parthenogenic stem cells; b) embryonic stem cells; c) inducible pluripotent stem cells; d) somatic cell nuclear transfer derived stem cells; e) Stimulus-triggered acquisition of pluripotency (STAP); and f) a combination thereof.

6. The conditioned media for use according to any one of claims 1-5, wherein the de-differentiated fibroblasts are produced upon exposure to hypoxia.

7. The conditioned media for use according to any one of claims 1-6, wherein the histone deacetylase inhibitor is selected from the group consisting of a) trichostatin A; b) phenylbutyrate; c) vorinostat; d) belinostat; e) LAQ824; f) panobinostat; g) entinostat; h) CI994; i) mocetinostat; j) sulforaphane; and k) a combination thereof.

8. The conditioned media for use according to any one of claims 4-7, wherein the de-differentiated fibroblasts are produced upon exposure to one or more DNA methyltransferase inhibitors.

9. The conditioned media for use according to claim 8, wherein the DNA methyltransferase inhibitor is selected from the group consisting of a) decitabine; b) 5-azacytidine; c) Zebularine; d) RG-108; e) procaine hydrochloride; f) Procainamide hydrochloride; g) Hydralazine hydrochloride; h) Epigallocatechin gallate; i) Chlorogenic acid; j) Caffeic acid; and k) a combination thereof.

10. The conditioned media for use according to any one of claims 4-9, wherein the de-differentiated fibroblasts are produced upon exposure to 2%-8%, 2%-7%, 2%-6%, 2%-5%, 2%-4%, 2%-3%, 3%-8%, 3%-7%, 3%-6%, 3%-5%, 3%-4%, 4%-8%, 4%-7%, 4%-6%, 4%-5%, 5%-8%, 5%-7%, 5%-6%, 6%-8%, 6%-7%, or 7%-8% oxygen.

11. The conditioned media for use according to any one of claims 2-10, wherein exosomes are obtained from de-differentiated fibroblasts.

12. The conditioned media for use according to claim 11, wherein the exosomes are purified from culture of the de-differentiated fibroblasts using anion exchange chromatography, high performance liquid chromatography (HPLC), or both.

13. The conditioned media for use according to any one of claims 1-12, the method comprising administering to the individual one or more of hyperbaric oxygen, adipose stem cell administration, bone marrow mesenchymal stem cell administration, fibroblast administration, and a combination thereof.

14. Exosomes for use in a method of treating or preventing disc degeneration in an individual, comprising the step of providing to the individual an effective amount of the exosomes.

15. The exosomes for use according to claim 14, wherein the exosomes are obtained from de-differentiated fibroblasts and/or conditioned media from de-differentiated fibroblasts, optionally wherein said de-differentiated fibroblasts are produced upon exposure to one or more histone deacetylase inhibitors.
